(19) **Europäisches Patentamt / European Patent Office / Office européen des brevets**

(11) **EP 1 286 691 B1**

(12) **EUROPEAN PATENT SPECIFICATION**

(45) Date of publication and mention
of the grant of the patent:
**14.11.2007 Bulletin 2007/46**

(21) Application number: **01927646.8**

(22) Date of filing: **02.05.2001**

(51) Int Cl.:
*A61K 38/34* *(2006.01)*    *A61K 38/22* *(2006.01)*

(86) International application number:
**PCT/DK2001/000304**

(87) International publication number:
**WO 2001/082953 (08.11.2001 Gazette 2001/45)**

(54) **METHODS FOR TREATMENT OF DISEASES ASSOCIATED WITH ACUTE INFLAMMATION UNDER NON-ISCHEMIC CONDITIONS**

VERFAHREN ZUR BEHANDLUNG VON ERKRANKUNGEN ASSOZIIERT MIT AKUTEN ENTZÜNDUNG UNTER NICHTISCHÄMISCHEN ZUSTÄNDE

METHODES DE TRAITEMENT DE MALADIES ASSOCIEES A UNE INFLAMMATION AIGÜE DANS LE CADRE DES PATHOLOGIES NON ISCHEMIQUES

(84) Designated Contracting States:
**AT BE CH CY DE DK ES FI FR GB GR IE IT LI LU MC NL PT SE TR**

(30) Priority: **02.05.2000 US 201264 P**
**11.08.2000 DK 200001204**
**22.11.2000 DK 200001757**
**06.03.2001 DK 200100369**

(43) Date of publication of application:
**05.03.2003 Bulletin 2003/10**

(73) Proprietor: **Action Pharma A/S**
**8200 Arhus N (DK)**

(72) Inventors:
• **NIELSEN, Soren**
**DK-8230 Abyhoj (DK)**
• **FROKIAER, Jorgen**
**DK-8230 Abyhoj (DK)**
• **JONASSEN, Thomas, Engelbrecht, Norkild**
**2840 Holte (DK)**

• **BJERKE, Thorbjorn**
**DK-3480 Fredensborg (DK)**

(74) Representative: **Plougmann & Vingtoft A/S**
**Sundkrogsgade 9**
**P.O. Box 831**
**2100 Copenhagen Ø (DK)**

(56) References cited:
**EP-A- 0 938 901**    **WO-A-88/00833**
**WO-A-96/14081**    **WO-A-98/10650**
**WO-A-99/52543**

• **JAMES M LIPTON ET AL: "Anti-inflammatory actions of the neuroimmunomodulator alpha-MSH" IMMUNOLOGY TODAY, vol. 18, no. 3, March 1997 (1997-03), pages 140-145, XP002902200**
• **ROBERT T. MEANS: "Erythropoietin in the treatment of anemia in chronic infectious, inflammatory, and malignant diseases." CURRENT OPINION IN HEMATOLOGY, vol. 2, 1995, pages 210-213, XP002902443 ISSN: 1065-6251**

## Description

### FIELD OF INVENTION

The present invention is defined in the claims and relates to the use of EPO optionally in combination with $\alpha$-MSH or an $\alpha$-MSH equivalent including the sequence His-Phe-Arg and acting on a melanocortin type 1 receptor for the preparation of a medicament for treatment or prevention of an acute inflammatory condition or disease under non-ischemic conditions in airways and lungs, or in the kidney or in the urinary tract and to a use according to any of claims 3-10, wherein EPO and $\alpha$-MSH or an $\alpha$-MSH equivalent including the sequence His-Phe-Arg and acting on a melanocortin type 1 receptor are to be administered independently of each other..

### BACKGROUND

### *Melanocortins*

Melanocortins are proopiomelanocortin-derived mammalian peptide hormones that include adrenocorticotropic hormone [ACTH (1-39)], $\alpha$- melanocyte-stimulating hormone [$\alpha$-MSH (1-13)], and related amino acid sequences including $\beta$- and $\gamma$-MSH. Melanocortin peptides have potent antiinflammatory/anticytokine activity (Lipton and Catania Immunol.Today, 18: 140-145, 1997). Melanocortins excert at least some of their effect via stimulation of melanocortin receptors. For melanocyte stimulating hormones (MSH) the action is in part ascribed to binding and activation of type 1-5 melanocortin receptors (MC1-MC5).

Melanocortins have a variety of functions including immunomodulation, anti-inflammation, body temperature regulation, pain perception, aldosterone synthesis, blood pressure regulation, heart rate, vascular tone, brain blood flow, nerve growth, placental development, synthesis/release of a variety of hormones such as aldosterone, thyroxin, prolactin, FSH. ACTH has a major effect on stimulating steroidoneogenesis. Also $\alpha$-MSH induces pigment formation in skin.

Five genes encoding melanocortin receptor subtypes have been identified (MC-receptor type 1-5). The MC receptors belong to the class of G-protein coupled receptors and have seven membrane spanning domains. All receptor subtypes involve increased production of camp to excert their actions. Type 2 receptor (MC2) represent the ACTH receptor whereas the other subtypes are melanocyto stimulating hormone receptors (MSH-receptors).

A series of studies have been performed on the MC receptors in a variety of tissues. Type 1 receptor (MC1), to which $\alpha$-MSH binds with great affinity, is known to be expressed in several tissues and cells such a brain, including astrocytes, testis, ovary, macrophages, neutrophils. However MC1 is likely to be expressed in an even wider range of tissues although this remains to be established. The selectivity for the MC=s to bind different melanocortin peptides vary. MC1 binds with great affinity $\alpha$-MSH and with lower affinity also $\beta$-MSH, $\gamma$-MSH and ACTH. MC2 has been reported only to bind ACTH but non of the MSH peptides. The highest affinity for the ligands of the other receptors including $\gamma$-MSH (MC3-receptor), $\beta$-MSH (MC4-receptor). In contrast MC5 binds with much lower affinity the MSH peptides but with the same pattern as MC1 (i.e. highest affinity for $\alpha$-MSH).

It is important to emphasize that a number of actions of MSH peptides, especially $\alpha$-MSH, are not fully established with respect to which receptors are involved. The anti-inflammatory action of $\alpha$-MSH has been speculated to involve a variety of processes including interference with NO production, endothelin-1 action, interleucin 10 formation, which again is linked to MC1 receptors expressed in macrophages, monocytes.

$\alpha$-MSH has also been shown to be important in a variety of inflamatory processes (Lipton and Catania 1997): 1) Inhibit chemotactive migration of neutrophils (Catania1996). 2) $\alpha$-MSH including analogs inhibit the release of cytokine (IL-1, TNF-$\alpha$) in response to LPS treatment (Goninard1996). 3) Inhibit TNF-$\alpha$ in response to bacterial endotoxin (Wong, K.Y. et al. Neuroimmunomodulation, 4: 37-41,1997). 4) ICV or IP administration of $\alpha$-MSH inhibit central TNF-$\alpha$ production by locally administered LPS. 5) $\alpha$-MSH has been shown to reduce the inflammation in experimental inflammatory bowel disease (Rajora, N. et al. Peptides, 18: 381-385, 1997), ishemia-induced acute renal failure(Star, R.A. et al. Proc.Natl.Acad.Sci.U.S.A, 92: 8016-8020, 1995). 6) $\alpha$-MSH also have some protective effect by inhibiting the induction and elicitation of contact hypersensitivity and induces hapten tolerance, and it is speculated that $\alpha$-MSH may mediate important negative regulation of cutaneous inflammation and hyper-proliferative skin diseases (Luger, T.A. J.lnvestig.Dermatol.Symp.Proc., 2: 87-93, 1997. To this end $\alpha$-MSH causes increased IL-8 release from dermal microvasculature endothelial cells (Hartmeyer, M. J.Immunol., 159: 1930-1937.1997).

### Erythropoetin (EPO)

The cellular adaptation to hypoxia involves many changes in gene expression, such as those of erythropoietin (Epo), vascular endothelial growth factor (VEGF), glycolytic enzymes, and tyrosine hydroxylase. Several reports have demonstrated that both oxygen sensing and chemical signaling occur via a common pathway that leads to the activation of hypoxia-inducible factor-1 (HIF-1), a transcription factor which is induced over a physiologically relevant range of oxygen tensions Epo is a 34-kDa glycoprotein hormone which has been characterized as the principal regulator of erythropoiesis and was thought to be exclusively produced in fetal liver and adult kidney in response to hypoxia. The molecular biology of the oxygen sensing mechanism underlying the transcriptional activity of Epo has been intensively investigated in HepG2 and Hep3B human hepatoma cell lines. In addition to transcriptional activation by HIF-1, mRNA stabilization has been found to account for an accumulation of Epo mRNA. Agents such as cobalt chloride (CoCl2) and desferrioxamine (DFX) are able to mimic the hypoxia-induced Epo transcription.

Indirect evidence has been provided to indicate that redox-mediated processes are likely to be involved in the induction of the EPO gene. Thus, iron and reactive oxygen species might play a critical role in the oxygen sensing mechanisms involved in the regulation of the expression of the EPO gene. Recent reports suggest that, along with its role in erythropoiesis, EPO might be of biological significance in the central nervous system. In vivo, EPO mRNA is expressed in both rodent and primate brain tissues and its expression is increased following hypoxia. Taken together, several findings imply that EPO acts on neurons in a paracrine way. This notion has been supported by the in vitro and in vivo europrotective effects of Epo. Several groups (Sadamoto, Y. et al. Biochem. Biophys. Res. Commun., 253: 26-32, 1998, Sakanaka, M. et al. Proc.Natl.Acad.Sci.U.S.A, 95:,4635-4640, 1998, Bernaudin, M. et al. J.Cereb.Blood Flow Metab, 19: 643-651, 1999) have shown that the direct administration of EPO to the central nervous system of mice, rats, and gerbils to some extent reduces neuronal death and prevents learning disability associated with cerebral ischemia.

BRIEF DESCRIPTION OF THE INVENTION

The effect of treatment with i.v. α-MSH equivalents alone, epoetin alone or α-MSH equivalents and epoetin combined was established in models of lung inflammation, systemic inflammation, kidney inflammation, and diseases of the urinary tract including the bladder. Models include LPS administration, ureteral obstruction, and aminoglycoside induced nephrotic syndrome. Various functional parameters were determined and the expression levels of relevant transporters were monitored to establish the effect of these compounds in these settings.

Acute/subacute LPS-inhalation induced lung and airway inflammation.
Treatment with the compounds significantly prevented the neutrophil and eosinophil airway and lung infiltration. Subacute systemic administration of LPS gave multiple organ inflammation including renal inflammation and treatment with the compounds significantly prevented the development of severe renal failure. Functional parameters of kidney function and of renal transporter expression were determined as parameters of treatment efficiency. Temporal ureteral obstruction for 24 hours (uni or bilateral) followed by release of obstruction for various time periods (1-30 days) induced significant changes in kidney and urinary tract function. Markers include functional parameters, downregulation of renal water and sodium transporters. Treatment with i.v. α-MSH -equivalents alone, epoetin alone or α-MSH and epoetin combined markedly reduced the downregulation of the renal marker proteins and prevented the reduction in kidney and urinary tract function.

In rat models of purimycin or adriamycin induced nephrotic syndrome ascites and proteinuria developed as prominent signs of severe nephritic syndrome. Other markers included downregulation of renal water channels (aquaporins) and sodium transporters. Treatment with i.v. α-MSH-equivalents alone, epoetin alone or α-MSH and epoetin combined markedly reduced the downregulation of the renal marker proteins and reduced proteinuria and ascites production.

Inflammation of airways and lung is often associated with swelling of airway and lung tissue and infiltration of airway and lung tissue with leucocytes including neutrophils, eosinophils or basophils and mast cells. This is well established and seen in extrodinary common conditions such as common cold, airway infection, and pulmonary infections, but also in conditions associated with allergy including allergic rhinitis, asthma or other allergic conditions. Moreover this is also seen in acute diseases associated with inflammation of lung and airways as well as in chronic obstructive pulmonary disease (COPD). At least three conditions contribute to COPD. (1) Chronic bronchitis is an inflammatory condition in which neutrophils, CD8+ T-lymphocytes and CD68+ monocytes/macrophages predominate. The condition is defined clinically by the presence of chronic cough and recurrent increase in bronchial secretion sufficient to to cause expectoration. There is enlargement of mucus-secreting glands and goble cell hyperplasia, which can occur in the absence of airflow limitation. (2) Adult chronic bronchiolitis is an inflammatory condition of small bronchi and bronchioli in which here are CD8+ and macrophages. (3) Emphysema is an inflammatory condition of the alveoli in which neutrophils, T-lymphocytes, and macrophages/monocytes are involved, associated with the release of excessive amounts of elastases from neutrophils. The course of COPD is characterized by intermittent exacerbations of the disease. In an axcerbation, there is also an significant influx of eosinophils into the tissue contributing to the inflammation. COPD is the fourth leading cause of death in the United States. The incidence, morbidity, and mortality of COPD is rising throught the world. The total economic cost of COPD in the US in 1993 was estimated to be over $US15.5 billion. Treatments effectively reducing or stopping the progress of COPD are needed. The ideal therapy in COPD would be compounds capable of reducing the influx of neutrophils into the airways or shrinking the enlarged mucous producing glands. As the inflammation continues natural repair processes start, resulting in peribronchial fibrosis, loss of lung elasticity and emphysema.

Also inflammation seen in other organs (either local) or attributed to systemic or more widespread inflammation is a feature associated with many diseases including infections, allergy, rheumatic diseases, cancer and other conditions and diseases or as a side-effect of drugs or poisoning.

In some cases of systemic inflammation this often involves inflammation or affection of the kidney leading to renal dysfunction or renal failure often seen in severe life-threatening conditions. The reduction in creatinine clearance or other renal functional parameters are indicative of such systemic inflammation.

Bilateral ureteral obstruction (BUO) is associated with reduction in renal functions. Characteristically, long-term loss of urinary concentration capacity is a common finding in both children and adults. The present inventors and others have shown that BUO and release of BUO in rats is associated with the onset of a dramatic postobstructive diuresis (POD) and a reduction in urinary concentrating capacity. Importantly, it was demonstrated that a BUO and release of BUO in rats was associated with a marked reduction in the protein expression of aquaporin-2 (AQP2), AQP3 and AQP1. Unilateral ureteral obstruction (UUO) is a model of renal injury characterized by progressive tubulointerstitial fibrosis and renal damage, while relatively sparing the glomerulus and not producing hypertension or abnormalities in lipid metabolism. Irrespective of the underlying cause, many kidney diseases lead to tubulointerstitial inflammation and eventual interstitial fibrosis with permanent loss of renal function. Most medical investigators agree that new therapeutic strategies should be targeted at developing effective methods for inhibiting renal fibrogenesis. The mechanisms responsible for UUO-induced kidney fibrosis are not well understood. However, prolonged obstruction induces progressive renal fibrosis with dysfunction, which cannot be readily restored even with removal of the obstruction. Under these circumstances, pharmacotherapeutic intervention needs to be developed to reverse or halt the progression of the renal dysfunction that occurs as a consequence of the obstruction. Furthermore, the development of progressive interstitial fibrosis represents a final common pathway associated with a variety of kidney disorders that can lead to functional insufficiency. Thus, the search for effective treatment preventing the progression is of great importance not only for elucidating the mechanism of UUO-induced fibrosis, but also for alleviating the renal fibrosis seen under various conditions with chronic renal failure (CRF).

UUO results in changes in renal hemodynamics, infiltration of the kidney by macrophages, and subsequent fibrosis of the tubulointerstitium. Many of the pathophysiological alterations associated with renal disease are driven by the intercrine, autocrine, paracrine, and endocrine effects of angiotensin II and it has been have demonstrated that angiotensin II production is rapidly stimulated following the onset of ureteral obstruction.

Angiotensin II, in turn, upregulates the expression of other factors including transforming growth factor- (TGF-), tumor necrosis factor- (TNF-$\alpha$), nuclear factor-B (NF-B), adhesion molecules, and chemoattractants, matrix proteins, and -smooth muscle actin (-SMA). The role of TNF- in the pathophysiology of obstructive uropathy, when compared with angiotensin II, is not well understood. In rats, pharmacological manoeuvers has been applied to inhibit angiotensin II formation or its biological action through receptor inhibition. No such pharmacological treatments are available to decipher the biological actions of TNF-. Two different cell surface receptors exist for TNF-, which are designated TNFR1 and TNFR2, that are derived from separate gene products. Moreover it has been shown that TNF- contributes, in part, to changes in interstitial volume, myofibroblast differentiation, and NF-B activation in the kidney during ureteral obstruction and are mediated through both the TNFR1 and TNFR2 gene products (mouse study). Thus the angiotensin II and TNF- systems appear to interact with each system, contributing to overall renal fibrosis. Also apoptosis plays a role and it has been shown that the expression of apoptotic and chemokine genes are significantly upregulated in UUO, and bioflavonoids and angiotensin inhibitors are able to attenuate the expression of these genes and thus may be beneficial in renal protection.

There are no really good treatment of these conditions: 1) The effect of anti-inflammatory treatment with corticosteroids (methylprednisolone) in patient with urinary tract obstruction caused by stones showed that treatment alone did not affect stone passage but combined treatment with the calcium antagonist treatment (nifedipine + methyl prednisolone) facilitated ureteral stone passage. In a few other studies it has been shown that corticosteroid treatment most likely is able to reduce the oedema of the ureteral wall associated with ureteral obstruction. 2) The effect of nonsteroidal anti-inflammatory treatment with cyclooxygenase inhibitors such as indomethacin, toradol and sulindic acid, agents which block prostaglandin synthesis, all have been shown to have some beneficial effect on stone passage and reduce pains associated with ureteral obstruction. The mechanism involved are speculated to be due to a direct effect on ureteral contraction, reduced oedema of the ureteral wall which in turn may reduce ureteral pressure. The effect to reduce pain by effecting ureteral pressure may also be due to a direct side effect on renal function where GFR and RBF are reduced due to blockade of the effects of vasodilating prostaglandins on renal hemodynamics. Thus a drug with major effect on renal and urinary tract function during obstruction and other kidney disorders associated with fibrosis etc is warranted.

Another common cause of renal failure is nephrotic syndrome, which is caused by glomerular damage and can be a result of treatment with drugs such as adriamycin or purimycin (PAN) aminoglycosides (and other drugs) but the underlying cause of most cases remains unidentified and many patients progress into renal failure (for references see). Nephrotic syndrome is associated with severe proteinuria, systemic edema including ascites, hypoproteinemia and hyperlipidemia. It is also associated with decreased urinary concentrating capacity and dilutional ability, and severe sodium and water retention is a cardinal feature in nephrotic syndrome leading to ascites, and progression into renal failure. The intrarenal factors leading to the dysregulation of kidney function are not well understood and currently there is no good treatment that prevents the progression of kidney damage. The glomerular changes and possibly also the tubular changes that results in glomerular and tubular dysfunction leading to heavy proteinuria, sodium and water retention and massive peripheral edema have been speculated to be secondary to infiltration with neutrophils, macrophages and monocytes (and induction of many cytokines and adhesion molecules), although this remains less well defined. Also insufficient proliferation and apoptosis in glomerular epithelial cells may be involved in the progression. Thus an inflammatory response is likely to contribute during some stages of the development of renal failure.

A number of renal transporters and channels have been shown to be dysregulated in association with nephrotic syndrome and this is likely to contribute to the derangement in kidney function. It has been speculated that the decrease in transporter expression in experimental nephritic syndrome is a direct effect of the causing agents (e.g. adriamycin or PAN) on the renal tuble and glomerual epithelial cells. The mechanism by which this agent produces obliteration of the foot processes of glomerular podocytes is not known, but the effect presumably involves impairment of the vesicle trafficking processes involved in maintaining the complex shape of these cells. It appears possible that the decline in ion transporter and water channel expression induced by adriamycin is a consequence of a similar impairment of trafficking in renal tubule cells although this is not established.

Nephrotic syndrome differs markedly from ishemia-induced acute renal failure, which is due to reduced or complete arrest in blood supply to the kidney(s). Nephrotic syndrome is often caused (as described above) by drugs (aminoglycosides and other antibiotics), infections, autoimmune disease, connective tissue diseases, cancer and many immune-mediated forms of glomerulonephritis and is never induced by ischemia. Ischemia is mainly associated with tubular damage whereas nephritic syndrome is mainly a glomerular disease with secondary affection of the tubular system. Thus the etiology is completely different.

As described above (and in the Lipton review) $\alpha$-MSH is likely to exert its effect via MC-1 receptors expressed in inflammatory cells (including neutrophils) or directly in the epithelium (not known). The effects of epoitin outside its effect in stimulating erythropoesis is virtually undefined but may include leads activation of hypoxia-induo-ible factor-1 (HIF-1), a transcription factor which is induced over a physiologically relevant range of oxygen tensions. EPO receptors have been found in multiple tissues including kidney and it is speculated that EPO acts via this receptor although binding to other receptors cannot be excluded.

DETAILED DESCRIPTION OF THE INVENTION

The present invention relates to the use of EPO optionally in combination with $\alpha$-MSH or an $\alpha$-MSH equivalent including the sequence His-Phe-Arg and acting on a melanocortin type 1 receptor for the preparation of a medicament for treatment or prevention of an acute inflammatory condition or disease under non-ischemic conditions in airways and lungs, or in the kidney or in the urinary tract. In the present context, the term medicament may accordingly represent either $\alpha$-MSH and/or $\alpha$-MSH equivalent as defined in claim 1 and/or EPO as well as any combination thereof. An example of a such combination is $\alpha$-MSH together with EPO.

By the term "an inflammatory condition" is in the present context meant a condition in which mechanisms such as reaction of specific T lymphocytes or antibody with antigen causes the recruitment of inflammatory cells and endogenous mediator chemicals. In some cases, the normal function of the organ or tissue will be altered by an increase in vascular permeability and/or by contraction of visceral smooth muscle. Such inflammatory conditions may give rise to inflammatory diseases.

Inflammatory diseases include the following diseases (non-limiting list): Arthritis, including diseases associated with arthritis), osteoartritis, rheumatoid arthritis; spondylarthropathies (e.g. ankylosing spondilitis), reactive arthritis (including arthritis following rheumatic fever), Henoch-Schonlein purpura, and Reiter's disease. Moreover inflammatory diseases include connective tissue disorders such as systemic lupus erythematosus, polymyositis/dermatomyositis, systemic sclerosis, mixed connetive tissue disease, polymyalgia rheumatica, and other types of vasculitis, crystal deposition diseases (including gout), pyrophosphate arthropathy, acute calcific periarthritis. Moreover inflammatory diseases include infective arthritis, juvenile arthritis (Still's disease), psoriasis, osteoarthritis, osteoarthritis secondary to hypermobilty, congenital dysplasias, slipped femoral epiphysis, Perthes disease, intra-articular fractures, meniscectomy, obesity, recurrent dislocation, repetitive actions, crystal depositions and diseases and meatobilic abnormalities of cartilage including pyrophosphate arthropathy, ochronosis, heamochromatosis, avascular necrosis including Sickle Cell disease, therapy with corticoids or other drugs, Caisson disease, septic or infections arthritis (including tuberculous arthritis, meningococcal arthristis, gonococcal arthritis, salmonella arthritis), Lymes disease, infective endocarditis (including endocarditis induced by Stretococcus viridans, Enterococcus Faecalis, Staphylococcus aureus, Staphylocossus epidermidis, Histoplasma, Brucella, Candida and Aspergellus species and Coxiella Bumetii), viral arthritis (including infection with rubella, mumps, hepatitis B, HIV or Parvovirus), or recurrent heamarthrosis. Moreover inflammatory diseases include connective tissue diseases such as systemic lupus erythematosus, polymyositis/dermatomyositis, systemic sclerosis, mixed connetive tissue disease, sarcoidosis and primary Sjogrens syndrome including keratoconjunctivitis sicca. Moreover inflammatory diseases include vasculitis such as infective vasculitis due to infections with bacterial species including spirochaetal diseses as Lyme disease, syphilis, rickettsial and mycobacterial infections, fungal, viral or protozoal infections. Moreover inflammatory diseases include non-infective vascultitis including Takayasu's arteritis, Giant Cell Arteritis (Temporal arteritis and polymyalgia rheumatica), Buerger's disease, polyarteritis nodosa, microscopic polyarteritis, Wegener's granulomatose, Churg-Strauss syndrome, Sarcoidosis, vasculitis secondary to connective tissue diseases including Systemic Lupus Erythematosus, Polymyositis/Dermatomyositis, Systemic Sclerosis, Mixed Connetive Tissue Disease, sarcoidosis and Primary Sjogrens syndrome. Moreover inflammatory diseases include vasculitis secondary to rheumatoid arthritis.

Moreover inflammatory diseases include non-infective vasculitis secondary to hypersensibility and leucocytoclastic vascultis including Serum Sickness, Henoch-Schonlein purpura, Drug induced vasculitis, essential mixed cryoglobulinaemia, hypocomplentaemia, Vascultis associated with other kinds of malignancy, inflammatory bowel disease and primary biliary cirrhosis, Goodpsture syndrome.

Moreover inflammatory diseases include all kinds of arthtitis in children such as Juvenile Chronic arthritis including Still's disease, juvenile rheumatoid arthritis, juvenile ankylosing spondylitis.

Moreover inflammatory diseases include all kinds of depostion diseases as Gout, pyrophosphate arthopathy and acute calcific periarthritis.

Moreover inflammatory diseases include all kind of inflammatory conditions causing backpain including infections, septic discitis, tuberculosis, malignancies (such as matastases, myeloma and others), spinal tumours, ancylosing spondylitis, acute disc prolapse, chronic disc diseaase/osteoarthritis, osteoporosis, and osteomalacia. It also includes Pagets disease, hyperparathyroidism, renal osteodystrophy, spondylolisthesis, spinal senosis congenital abnormalities and fibromyalgia.

Moreover inflammatory diseases include all kinds of soft-tissue rheumatism including bursitis, tenosynovitis or peritendonitis, enthesitis, nerve compression, periarthritis or capsulitis, muscle tension and muscle dysfunction.

Moreover inflammatory diseases include inflammatory diseases of the gastrointestinal system (including stomatitis of all kinds, pemfigus, bulloid pemphigoid and benig mucous membrane pemphigoid), salivary gland diseases (such as sarcoidosis, salivary duct obstruction and Sjogrens syndrome), inflammaton of the oesophagus (e.g. due to gastrooesophagel reflux or infections with candida species, herpes simplex and cytomegalus virus), inflammatory diseases of the stomach (including acute and chronic gastritis, helicobacteer pylori infection and Mentriers disease), inflammation of the the small intestine (including coeliac disease, gluten sensitive enteropathy, dermatitis herpitiformis, tropical sprue, Whipple's diease, radiation enteritis, systemic amyloidosis, connective tissue disorders including systemic lupus erythematosus, pofymyositis/dermatomyositis, systemic sclerosis, mixed connetive tissue disease and sarcoidosis), eosinophilic gastroenteritis, intestinal lympangiectasia, inflammatory bowel disease (including Chrons disease and ulcertive colitis), diverticular disease of the colon, and irritable bowel syndrome

Moreover, inflammatory diseases include upper and lower airway diseases such as chronic obstructive pulmonary diseases (COPD), allergic and non-allergic asthma, allergic rhinitis, allergic and non-allergic conjunctivitis. Moreover, inflammatory diesases also include allergic and non-allergic dermatitis.

The treatment of inflammatory conditions or diseases under non-ischemic conditions, i.e. conditions wherein there is substantially normal blood supply to the organ or organs in questions is described.

One preferred target organ is airways and lungs known to be site for inflammation in acute respiratory diseases. Another preferred target organ is the kidney, including tubules and glomeruli and the urinary tract system comprising ureteres, bladder, and urethra. However, also other cell types such as the prostate may be involved in an inflammatory condition.

The cells to be treated may be one or more cell types selected from macrophages, the reticulo endothelial system monocytes, neutrophil granulocytes, eosinophil granulocytes, basophil granulocytes, T-cells, B-cells, mast cells, and dendritic cells.

In its broadest concept the description relates to any condition wherein the normal function of the organs or tissues is altered including conditions associated with ischemia, acute and/or chronic inflammation, allergy, rheumatic diseases, infection including viral, fungal, bacterial infections, prions and other microbes and infectious agents known in the art. The injury may include acute and chronic injury. Chronic injury includes situations of repetitive injuries alternating with periods of complete or partial recovery of the organ(s) or tissue(s) function. The invention also relates to injury, which is associated with implantation of one or more organs or other devices for transplantation. The organ can be from the individual him or herself, the animal itself or from other individuals or animals. This includes: organ transplants, bone transplants, soft tissue implants (silicone implants), metal and plastic implants, or other medical implantable devices. Individual represents humans as well as other mammals.

The condition to be treated may be caused by a cancer or a by premalignant disorder having an impact on the organ, e.g. on the respiratory system including lung, bronchiole, upper airways, and/or on the heart and/or on the kidney and/or on the gastrointestinal system, including acute leukemia, chronic myelocytic leukemia, chronic lymphocytic leukemia, Hodgkin's disease, lymphosarcoma, myeloma, metastasizing carcinoma of any origin.

The condition to be treated may be caused by any disease selected from diabetes mellitus, conditions with increased fasting levels of LDL-Cholesterol, conditions with combined increased fasting levels of LDL-Cholesterol and triglycerid, conditions with increased fasting levels of triglycerid, conditions with increased fasting levels of HDL-Cholesterol, retroperitoneal fibrosis, lupus erythematosus, polyarteritis nodosa, sclerodermia, polymyositis, dermatomyositis, rheumatoid arthritis, anaphylaxis, serum sickness, hemolytic anaemia, and allergic agranulocytosis.

Many infections may have an influence on the tissue and disturb the normal function resulting in decreased performance which may be improved by administration of an effective dose of α-MSH and/or an α-MSH equivalent and EPO and/or an EPO equivalent. Such infections include infections by protozoa, virus, bacteria and fungus and include conditions such as AIDS, bacterial septicemia, systemic fungal infections, Rickettsial diseases, toxic shock syndrome, infectious mononucleosis, chlamydia thrachomatis, chlamydia psittaci, cytomegalovirus infection, campylobacter, salmonella, influenza, poliomyelitis, toxoplasmosis, Lassa Fever, Yellow Fever, billharziose, colibacteria, enterococcus, preteus, klebsiella, pseudomonas, staphylococcus aureus, staphylococcus epidermidis, candida albicans, tuberculosis, mumps, infectious mononucleosis, hepatitis and Coxackie virus

The condition to be treated may be associated with a chemical trauma involving one or more toxic substances and/or drugs. Such drugs include tricyclic antidepressants, lithium salts, prenylamine, phenothizine derivatives, chemopreventive drugs including adriamycin. Also physical traumas including electromagnetic radiation may cause damages which can be alleviated by administration of an effective dose of an α-MSH and/or of an α-MSH equivalent and/or administration of an EPO and/or an EPO equivalent according to the present invention.

The condition, which may be treated, may further include connective tissue disease such as scleroderma, systemic lupus erythematosus or by neuromyopathic disorders such as progressive muscular dystrophy of Duchenne's type, Friedreich's ataxia, and myotonic dystrophy. The condition may e.g. be related to the tissue of the intestine of the mammal.

The medicament in question may be administered therapeutically for treating an existing condition or prophylactically for preventing a progress of the condition, or of any symptom of the condition. It may be administered prophylactically for preventing the establishment of the condition or of any symptom of the condition. The α-MSH and/or α-MSH equivalent and/or rh-EPO and/or a rh-EPO equivalent may be administered as a single dosage, as continued administration including a regimen where specific dosages are prescribed for a shorter or longer duration, or as a sequential administration similarly with many treatment schedules for cancer therapy.

The origin of the condition may include anatomic abnormality of the tissue or organ, inflammatory diseases, and/or conditions caused by a chemical trauma including drugs such as adriamycin and other chemotherapeutics; electromagnetic radiation; renal and/or ureteric calculi, especially when the calculi occur frequently.

The preferred target organ according to the invention is airways, lung, ureteres, kidney, bladder, urethra and the prostate gland and wherein the tissue(s) is selected from the group of lymphoid tissues, mucosa, epithelium, and endothelium. However also inflammation in other organ systems or parts of organs may be succesfully treated. Examples of such organs or organ systems which may be treated are skin, skeleton, brain and central nervous system, muscles, vessels, upper and lower respiratory tract, lungs and pleura, exocrine and endocrine glands, heart and pericardium, intestinal tract including excretory ducts of liver and pancreas, liver, pancreas, genital organs and uterus, kidney and urinary tract including prostate.

The treatment and/or prevention of infections in target organs including lung and airways but also cystitis, especially interstitiel cystitis, and cystitis limited to the mucosa is also described. The infections and inflammation may be a microbial or non-microbial. The infection or inflammation may be local or in distinct organ regions or systemic.

Many diseases such as rheumatic diseases, retroperitoneal fibrosis, lupus erythematosus, polyarteritis nodosa, sclerodermia, polymyositis, dermatomyositis, rheumatoid arthritis, anaphylaxis, serum sickness, hemolytic anaemia, allergic agranulocytosis, may succesfully be treated.

Another important embodiment is the conditions wherein diabetes mellitus is involved.

Yet another important embodiment is conditions associated with inflammation of skin.

Examples of conditions to be treated by the methods according to the present invention is wherein the condition is lung and/or airways, kidney (recognized as renal failure, nephrotic syndrome) or complete or partial urinary tract obstruction, postoperative polyuria.

In one important embodiment the condition to be treated is associated with inflammation of lung and airways with infiltration of one or more of leucocytes including neutrophils, eosinophils, lymphocytes and monocytes but also macrophages, mast cells or basophils. These conditions include chronic obstructive pulmonary diseases with or without acute infections or worsening of inflammation, allergic diseases, astma. This also includes inflammation in the airways including upper airways caused by allergy or infection or other diseases.

In one embodiment the condition is associated with reduced renal function indicated by one or more of the following conditions; reduced renal blood flow, reduced glomerular filtration rate, reduced urinary concentrating ability, reduced urinary concentration capacity, reduced or increased urinary electrolyte excretion (such as sodium, potassium, bicarbonate), reduced creatinine clearance which may be treated or prevented by the methods according to the invention.

Accordingly, the condition may be associated with dysregulation of one or more renal sodium transporters. Such downregulation of one or more renal sodium transporters may be of at least 50 %, such as at least 75%, compared to non-treatment. The sodium transporters may be selected from the group consisting of Na,K-ATPase, NHE-3, NaPi-2, BSC-1, TSC and ENaC's.

Also dysregulation of one or more renal aquaporins may characterize a condition to be treated according to the invention including downregulation of one or more renal aquaporins including aquaporins selected from aquaporins 1 to 12, preferably aquaporins 1 to 4.

The administration according to the use of the present invention may be any administration known in the art as may easily be recognised by the skilled person according to the individual situation. Accordingly, the administration may be selected from systemic administration; injection into tissue or into a to body cavity including joints; implantation into tissue or into a body cavity; topical application to the skin or to any gastrointestinal surface, or to a mucosal surface including the lining of body cavities.The administration may be selected from parenteral adminstration, including intraperitonal administration, intrathecal administration systemic administration, local administration, topical administration, transmucosal administration, transdermal administration and oral administration.

The α-MSH equivalent according to the present invention is a substance acting on an α-MSH receptor and/or on a melanocortin receptor such as subtypes 1 to 5 (MC-receptors 1-5). Such substances are disclosed in e.g. EP 972522, WO 87/04623, WO 88/00833, WO 99/57148, WO 99/21571, WO 96/41815, US 5028592, US 5,731,408, US 5,830,994 and the references cited therein.

The α-MSH equivalent is a polypeptide having at least 3 amino acids including the following sequence Lys-Pro-Val, such as Gly-Lys-Pro-Val, or the following sequence His-Phe-Arg, and being able to act on an α-MSH receptor.

The treatment or prevention as described above is in a preferred embodiment performed with EPO. In this respect, the effective dosage of the unit of EPO is lower than the dosage in which EPO is generally used for its known indications. The necessary dosage of EPO will generally be a completely non-toxic dosage for the individual.

By use of the combination of an α-MSH or α-MSH equivalent with rh-EPO, a synergistic effect may be obtained. The synergism may during a treatment period be at least 5% or even higher such as at least 10%, preferebly at least 15% as measured according to the selected test system in an organ. It is believed that a synergistic effect of at least 20%, such as at least 25%, may be demonstrated by a treatment according to the invention. The test system may be any of the experimental protocols described herein.

Pharmaceutical formulations and compositions:

In the following examples of suitable compositions containing α-MSH and/or an α-MSH equivalent and EPO are given. Depending on the use of the α-MSH and/or an α-MSH equivalent and EPO, a composition may be a pharmaceutical or a cosmetic composition.

For the administration to an individual (an animal or a human) the substance(s) are preferably formulated into a pharmaceutical composition containing the substance(s) and, optionally, one or more pharmaceutically acceptable excipients.

The compositions may be in form of, e.g., solid, semi-solid or fluid compositions such as, e.g., but not limited to bioabsorbable patches, drenches, dressings, hydrogel dressings, hydrocolloid dressings, films, foams, sheets, bandages, plasters, delivery devices, implants,

powders, granules, granulates, capsules, agarose or chitosan beads, tablets, pills, pellets, microcapsules, microspheres, nanoparticles, sprays, aerosols, inhalation devices,

gels, hydrogels, pastes, ointments, creams, soaps, suppositories, vagitories, tooth paste,solutions, dispersions, suspensions, emulsions, mixtures, lotions, mouthwash, shampoos, enemas,

kits containing e.g. two separate containers, wherein the first one of the containers contains the α-MSH and/or α-MSH equivalent and/or EPO and/or pharmaceutically acceptable excipients and the second container containing a suitable medium intended to be added to the first container before use in order to obtain a ready-to-use composition;and in other suitable forms such as, e.g., implants or coating of implants or in a form suitable for use in connection with implantation or transplantation.

The compositions may be formulated according to conventional pharmaceutical practice, see, e.g., "Remington: The science and practice of pharmacy" 20th ed. Mack Publishing, Easton PA, 2000 ISBN 0-912734-04-3 and "Encyclopedia of Pharmaceutical Technology", edited by Swarbrick, J. & J. C. Boylan, Marcel Dekker, Inc., New York, 1988 ISBN 0-8247-2800-9.

A pharmaceutical composition comprising an active substance serves as a drug delivery system. In the present context the term "drug delivery system" denotes a pharmaceutical composition (a pharmaceutical formulation or a dosage form) which upon administration presents the active substance to the body of a human or an animal. Thus, the term "drug delivery system" embraces plain pharmaceutical compositions such as, e.g., creams, ointments, liquids, powders, tablets, etc. as well as more sophisticated formulations such as sprays, plasters, bandages, dressings, devices, etc.

Apart from α-MSH and/or an α-MSH equivalent and/or EPO, a pharmaceutical composition for use according to the invention may comprise pharmaceutically or cosmetically acceptable excipients.

The choice of pharmaceutically acceptable excipients in a composition for use according to the invention and the optimum concentration thereof cannot generally be predicted and must be determined on the basis of an experimental determination thereof. Also whether a pharmaceutically acceptable excipient is suitable for use in a pharmaceutical composition is generally dependent on which kind of dosage form is chosen. However, a person skilled in the art of pharmaceutical formulation can find guidance in e.g., "Remington: The science and practice of pharmacy" 20th ed. Mack Publishing, Easton PA, 2000 ISBN 0-912734-04-3.

A pharmaceutically acceptable excipient is a substance, which is substantially harmless to the individual to which the composition will be administered. Such an excipient normally fulfils the requirements given by the national drug agencies. Official pharmacopeias such as the British Pharmacopeia, the United States of America Pharmacopeia and the European Pharmacopeia set standards for well-known pharmaceutically acceptable excipients.

In the following is given a review on relevant pharmaceutical compositions for use according to the invention. The review is based on the particular route of administration. However, it is appreciated that in those cases where a pharmaceutically acceptable excipient may be employed in different dosage forms or compositions, the application of a particular pharmaceutically acceptable excipient is not limited to a particular dosage form or of a particular function of the excipient.

Parenteral compositions:

For systemic application, the compositions according to the invention may contain conventionally non-toxic pharmaceutically acceptable carriers and excipients according to the including microspheres and liposomes.

The compositions for use according to the invention include all kinds of solid, semisolid and fluid compositions. Compositions of particular relevance are e.g. solutions, suspensions, emulsions, gels, implantation tablets and implants.

The pharmaceutically acceptable excipients may include solvents, buffering agents, preservatives, humectants, chelating agents, antioxidants, stabilizers, emulsifying agents, suspending agents, gel-forming agents, diluents, disintegratig agents, binding agents, lubricants and wetting agents. For examples of the different agents see below.

Topical, trans-mucosal and trans-dermal compositions:

For application to the mucosa or the skin, the compositions for use according to the invention may contain conventionally non-toxic pharmaceutically acceptable carriers and excipients including microspheres and liposomes.

The compositions for use according to the invention include all kinds of solid, semi-solid and fluid compositions. Compositions of particular relevance are e.g. pastes, ointments, hydrophilic ointments, creams, gels, hydrogels, solutions, emulsions, suspensions, lotions, liniments, resoriblets, suppositories, enema, pessaries, moulded pessaries, vaginal capsules, vaginal tablets, shampoos, jellies, soaps, sticks, sprays, powders, films, foams, pads, sponges (e.g. collagen sponges), pads, dressings (such as, e.g., absorbent wound dressings), drenches, bandages, plasters and transdermal delivery systems.

The pharmaceutically acceptable excipients may include solvents, buffering agents, preservatives, humectants, chelating agents, antioxidants, stabilizers, emulsifying agents, suspending agents, gel-forming agents, ointment bases, suppositoriy bases, penetration enhancers, perfumes, skin protective agents, diluents, disintegratig agents, binding agents, lubricants and wetting agents. For examples of the different agents see below.

Oral compositions:

For application to the mucosa or the skin, the compositions for use according to the invention may contain conventionally non-toxic pharmaceutically acceptable carriers and excipients including microspheres and liposomes.

The composition for use according to the invention include all kinds of solid, semi-solid and fluid compositions. Compositions of particular relevance are e.g. solutions, suspensions, emulsions, uncoated tablets, modified-release tablets, gastro-resistant tablets, orodispersible tablets, efferverscent tablets, chewable tablets, soft capsules, hard capsules, modified release capsules, gastro-resistant capsules, uncoated granules, effervescent granules, granules for the preparation of liquids for oral use, coated granules, gastro-resistant granules, modified-release granules, powders for oral administration and powders for the preparation of liquids for oral use.

The pharmaceutically acceptable excipients may include solvents, buffering agents, preservatives, humectants, chelating agents, antioxidants, stabilizers, emulsifying agents, suspending agents, gel-forming agents, diluents, disintegratig agents, binding agents, lubricants, coating agents and wetting agents. For examples of the different agents see below.

Examples of various agents:

Examples of solvents are but not limited to water, alcohols, vegetable or marine oils (e.g. edible oils like almond oil, castor oil, cacao butter, coconut oil, com oil, cottonseed oil, linseed oil, olive oil, palm oil, peanut oil, poppyseed oil, rapeseed oil, sesame oil, soybean oil, sunflower oil, and teaseed oil), mineral oils, fatty oils, liquid paraffin, polyethylene glycols, propylene glycols, glycerol, liquid polyalkylsiloxanes, and mixtures thereof.

Examples of buffering agents are but not limited to citric acid, acetic acid, tartaric acid, lactic acid, hydrogenphosphoric acid, diethylamine etc.

Examples of preservatives for use in compositions are but not limited to parabens, such as methyl, ethyl, propyl p-hydroxybenzoate, butylparaben, isobutylparaben, isopropylparaben, potassium sorbate, sorbic acid, benzoic acid, methyl benzoate, phenoxyethanol, bronopol, bronidox, MDM hydantoin, iodopropynyl butylcarbamate, EDTA, benzalconium chloride, and benzylalcohol, or mixtures of preservatives.

Examples of humectants are but not limited to glycerin, propylene glycol, sorbitol, lactic acid, urea, and mixtures thereof.

Examples of chelating agents are but not limited to sodium EDTA and citric acid.

Examples of antioxidants are but not limited to butylated hydroxy anisole (BHA), ascorbic acid and derivatives thereof, tocopherol and derivatives thereof, cysteine, and mixtures thereof.

Examples of emulsifying agents are but not limited to naturally occurring gums, e.g. gum acacia or gum tragacanth; naturally occurring phosphatides, e.g. soybean lecithin; sorbitan monooleate derivatives; wool fats; wool alcohols; sorbitan esters; monoglycerides; fatty alcohols;, fatty acid esters (e.g. triglycerides of fatty acids); and mixtures thereof.

Examples of suspending agents are but not limited to celluloses and cellulose derivatives such as, e.g., carboxymethyl cellulose, hydroxyethylcellulose, hydroxypropylcellulose, hydroxypropylmethylcellulose, carraghenan, acacia gum, arabic gum, tragacanth, and mixtures thereof.

Examples of gel bases and viscosity-increasing are but not limited to liquid paraffin, polyethylene, fatty oils, colloidal silica or aluminium, zinc soaps, glycerol, propylene glycol, tragacanth, carboxyvinyl polymers, magnesium-aluminium silicates, Carbopol®, hydrophilic polymers such as, e.g. starch or cellulose derivatives such as, e.g., carboxymethylcellulose, hydroxyethylcellulose and other cellulose derivatives, water-swellable hydrocolloids, carragenans, hyaluronates (e.g. hyaluronate gel optionally containing sodium chloride), and alginates including propylene glycol aginate.

Examples of ointment bases are but not limited to beeswax, paraffin, cetanol, cetyl palmitate, vegetable oils, sorbitan esters of fatty acids (Span), polyethylene glycols, and condensation products between sorbitan esters of fatty acids and ethylene oxide, e.g. polyoxyethylene sorbitan monooleate (Tween).

Examples of hydrophobic ointment bases are but not limited to paraffins, vegetable oils, animal fats, synthetic glycerides, waxes, lanolin, and liquid polyalkylsiloxanes.

Examples of hydrophilic ointment bases are but not limited to solid macrogols (polyethylene glycols).

Examples of powder components are but not limited to alginate, collagen, lactose, powder which is able to form a gel when applied to a wound (absorbs liquid/wound exudate).

Examples of diluents and disintegrating agents are but not limited to lactose, saccharose, emdex, calcium phosphates, calcium carbonate, calcium sulphate, mannitol, starches and microcrystaline cellulose.

Examples of binding agents are but not limited to saccharose, sorbitol, gum acacia, sodium alginate, gelatine, starches, cellulose, sodium coboxymethylcellulose, methylcellulose, hydroxypropylcellulose, polyvinylpyrrolidone and polyetyleneglycol.

Examples of wetting agents are but not limited to sodium laurylsulphate and polysorbate 80.

Examples of lubricants are but not limited to talcum, magnesium stearate, calcium stearate, silicium oxide, precirol and polyethylenglycol.

Examples of coating agents are but not limited to hydroxypropylcellulose, hydroxypropylmethylcellulose, polyvinylpropylidone, ethylcellulose and polymethylacrylates.

Examples of suppository bases are but not limited to oleum cacao, adeps solidus and polyethylenglycols.

The $\alpha$-MSH and/or $\alpha$-MSH equivalent and/or EPO is present in the medicament in an amount of 0.001-99%, typically 0.01-75%, more typically 0.1-20%, especially 1-10% by weight of the medicament.

The EPO and/or EPO equivalent may be present in the medicament in an amount of 0.001-99%, typically 0.01-75%, more typically 0.1-20%, especially 1-10% by weight of the medicament. The EPO and/or EPO equivalent is generally used in dosages, which are completely non-toxic to a human. By use of a combination of an EPO together with a unit dosage of $\alpha$-MSH and/or an $\alpha$-MSH equivalent, an effect of the combination may be obtained which is higher than the effect obtained with any of the substances administered alone.

With respect to the combination, it is possible to obtain an synergistic effect where the EPO and the $\alpha$-MSH

and/or α-MSH equivalent is administered independently of each other. The time span from the release of one of the active ingredients to the organ or tissue in question until the other active ingredient is subjected to the tissue or organ may be several days, even 5 days or one week. However, the drugs are preferable administered within about 48 hours, preferably within 24 hours, such as within 12 hours. However, for practical reasons the active ingredients will normally be substantially co-administered with considerations to differences in pharmacokinetic properties and speed-of-action of the compounds.

In a further embodiment the present invention relates to a pharmaceutical composition comprising a unit dosage of EPO and a unit dosage of α-MSH and/or of an α-MSH equivalent, optionally together with a suitable pharmaceutical carrier. The carrier may be selected according to the specific use as disclosed above. In a further aspect, the composition may be specifically adapted for any of the uses and methods disclosed herein.

It is contemplated that the dose of α-MSH and/or α-MSH equivalent will be in the range of 1 ng to 100 mg pr. kg body weight, typically 1µg to 10 mg pr. kg body weight, more typically 10µg to 1 mg pr. kg body weight, such as 50-500 µg pr. kg body weight; and that the dose of EPO and/or EPO equivalent will be in the range of 0,001-10000 IU pr. kg body weight, typically 0,1-5000 IU pr. kg body weight, more typically 1-1000 IU pr. kg body weight, such as 50-500 IU pr. kg body weight.

A further aspect of the invention is a pharmaceutical kit comprising a unit dosage of EPO and a unit dosage of α-MSH and/or of an α-MSH equivalent optionally together with a suitable pharmaceutical carrier and optionally a description of the specific use. The kit may comprise the α-MSH equivalent or α-MSH in any of the forms described herein and the EPO may be in an identical for or in any other form. Accordingly, the α-MSH may be present in a device for sustained effect whereas the EPO may be present in the kit in a form suitable for injection. The specific kit may accordingly be designed for the individual treatment of prophylactic use.

LEGEND TO FIGURES

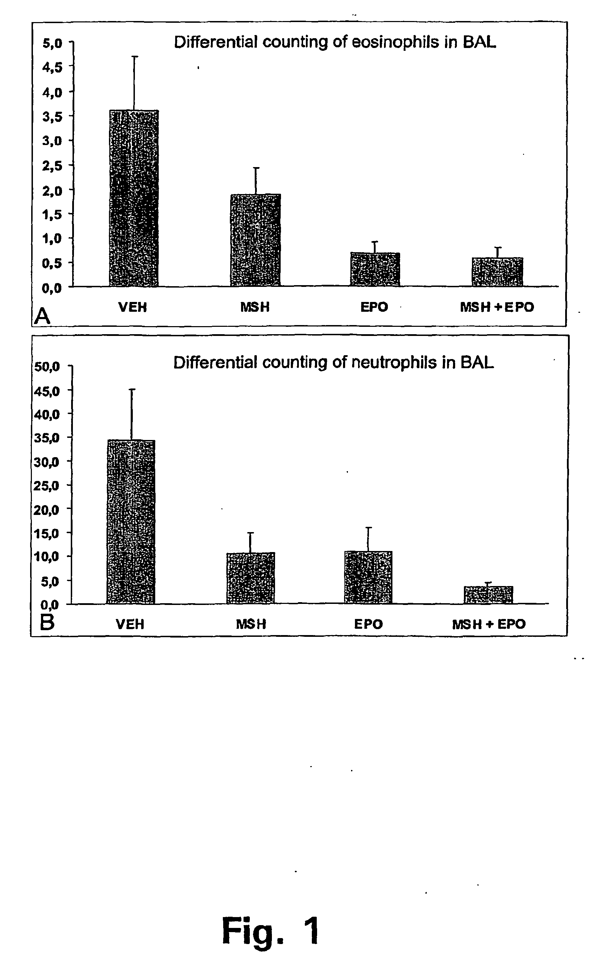

Fig 1. Differential counting showed a marked reduction in the number of inflammatory cells in the lungs with all three treatment regimens. Panel A: The number of eosinophils were significantly reduced with EPO treatment and combination treatment with α-MSH+EPO. Panel B: The data show a dramatic reduction in the neutrophils in response to combined treatment with α-MSH+EPO.

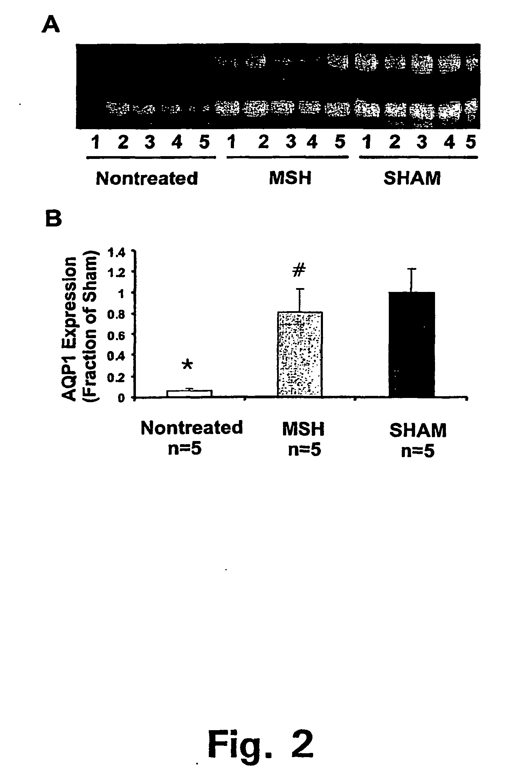

Fig 2. Panel A shows an immunoblot from whole kidney reacted with affinity-purified anti-aquaporin-1 (anti-AQP1) which revealed 29 kDa and 35-50 kDa AQP1 bands, representing non-glycosylated and glycosylated forms of AQP1. Panel B shows densitometric analysis. In response to 24 hours of BUO and 5 hours release densitometric analysis of all samples from nontreated, α-MSH treated rats with 24-hour BUO and sham-operated controls revealed that AQP1 expression decreased from $100\pm22\%$ in sham operated controls to $7\pm2\%$ in BUO rats without α-MSH treatment,* $P<0.05$. α-MSH treatment significantly increased the level of AQP1 expression to $81\pm21\%$ compared with nontreated rats with BUO, # $P<0.05$. Thus α-MSH treatment prevented the dramatic decrease in AQP1 expression in response to 24 hours of BUO and 5 hours of release.

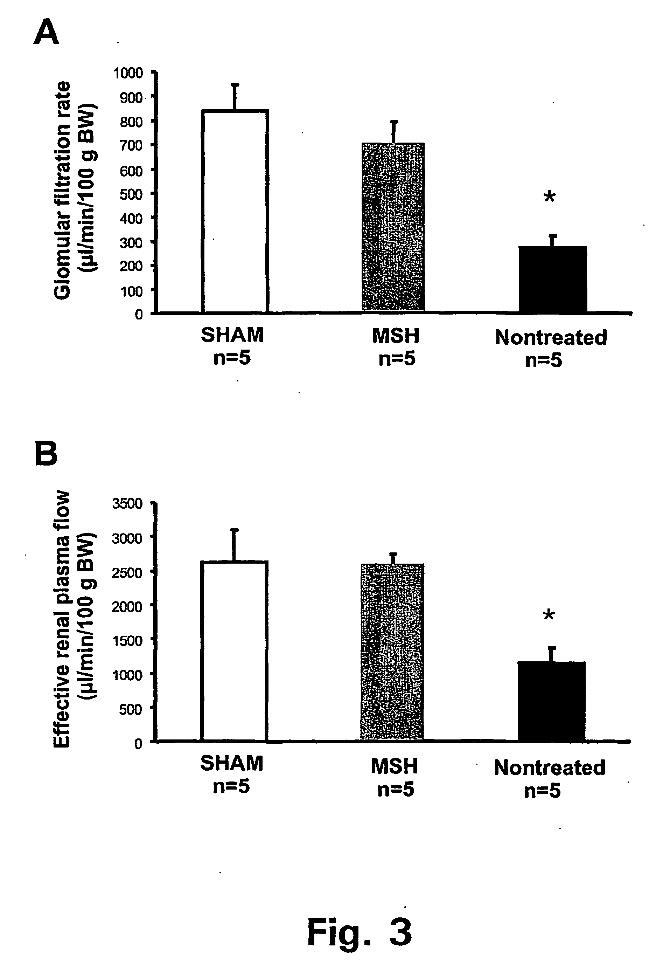

Fig 3. Panel A: Glomerular filtration rate (GFR) did not differ among the three groups at baseline level. α-MSH treatment completely prevented the reduction in GFR (BUO+MSH: $705 \pm 85$ µl/min/100g vs. SHAM: $840 \pm 105$ µl/min/100g) 48 hours after release of BUO. Panel B: Effective renal plasma flow (ERPF) did not differ among the three groups at baseline level. α-MSH treatment completely prevented the reduction in ERPF (BUO+α-MSH: $2598 \pm 129$ µl/min/100g vs. SHAM: $2633 \pm 457$ µl/min/100g) 48 hours after release of BUO.

EXAMPLES

*Experimental animals*

Studies were performed on adult male Munich Wistar rats or Spraque Dawley rats (Møllegard Breeding centre Ltd., Eiby, Denmark). The rats were maintained on a standard rodent diet (Altromin, Lage, Germany) with free access to water. During the entire experiment, rats were kept in individual metabolic cages, with a 12:12h artificial light/dark cycle, a temperature of 21°C, plus/minus 2°C. Rats were allowed to acclimatize to the cages for 3 days prior to surgery.

*induction of acute lung inflammation*

Studies were performed on male Spraque Dawley rats (Møllegard Breeding centre Ltd., Eiby, Denmark). The rats were maintained on a standard rodent diet (Altromin, Lage, Germany) with free access to water. Inflammation of the lungs (asthma) was induced by LPS inhalation with the animals anesthetized using Midazolam and Hypnorm The LPS was administrated by connecting the rat to a semiclosed breathing system consisting of a MAXIN bottle filled with a mixture of LPS in saline connected to a standard inhalation chamber. Each animals is exposed for 20 min with 5 mg LPS. Twenty-four hours after exposure to LPS rats were killed. Subsequently, lavage was performed by washing the lungs with PBS (5 ml) 6 times. The lung tissue was then centrifuged for 10 min (1000 rpm) and resuspended in 0.5 ml. From each sample 2 blood smears were made. They were air-dried, fixed and counterstained for differential cell counting.

*Induction of acute systemic inflammation*

Barrier-bred and specific pathogen-free female Wistar rats (210-230 g) were obtained from the Department of Experimental Medicine, Panum Institute, University of Copenhagen, Denmark. The animals were housed in a temperature (22-24° C) and moisture (40-70%) controlled room with a 12-hour light-dark cycle (light on from 6:00 A.M. to 6:00 P.M.). All animals were given free access to tap water and a pelleted rat diet containing approximately 140 mmol/kg of sodium, 275 mmol/kg potassium and 23 % protein (Altromin catalogue no. 1310, Altromin International, Lage, Germany). Rats were anesthetized with halothane-nitrous oxide and permanent medical grade Tygon catheters were implanted into the abdominal aorta and into the inferior caval vein via a femoral artery and vein. After instrumentation, the animals were housed individually. All surgical procedures were performed during aseptic conditions. To relieve postoperative pain, rats were treated with buprenorfin, 0.2 mg/kg body weight i.p. Two weeks later the rats were anesthetized with halothane-nitrous oxide and a osmotic minipump (Alzet 1003D)filled with a LPS solution was implanted into the abdomen. The mean infusion rate of LPS was 200 μg/kg/hour. All rats except the control rats received LPS infusion. N=5 in all groups.

*Induction of bilateral ureteral obstruction*

Adult Munich-Wistar rats were anesthetized with halothane and placed on a heating board under an operating microscope, Through a midline abdominal incision both ureters were exposed and occluded by placing a 5-mm piece of bisected polyethylene tubing (PE-50) around the midportion of each ureter. The ureter was then occluded by tightening the tubing with a 5-0 silk ligature. 24 hours later, the obstructed ureters were decompressed by removal of the ligature and the PE-50 tubing. Done in this manner, both ureters could be completely occluded for 24 h and without evidence of functional impairment of ureteral function and rats followed up to several days after release of obstruction.

*Induction of unilateral ureteral obstruction*

Adult Munich-Wistar rats were anaesthetized with intraperitoneal sodium pentobarbital (50 mg/kg) and placed on a heating board under an operating microscope. Through a midline abdominal incision the left ureter was exposed and a 5 mm piece of bisected polyethylene tubing (PE-50) was placed around the midportion of the ureter. The ureter was then occluded by tightening the tubing with a 5-0 Silk ligature. Twenty-four hours later, the obstructed ureters were decompressed by removal of the ligature and the PE tubing. Using this technique the ureter could be completely occluded for 24 hours without evidence of subsequent functional impairment of ureteral function.

*Induction of purimycin and adriamycin-induced nephrotic syndrome in rats*

Nephrotic syndrome was induced by a single i.p. or s.c. injection of adriamycin or purimycin (at various doses) and the rats were followed for 7 days - 21 days.
Development of severe nephrotic syndrome was established by monitoring the total body weight, urinary protein excretion (proteinuria) and at time of ending the experiment the volume of fluid in the abdominal cavity was determined (rats develop severe water retention and ascites). The effect of α-MSH or epoetin or α-MSH combined with epoetin was determined by treatment with the compounds for the initial 3 days after adrimycin or purimycin administration.

*Experimental protocols*

The following protocols were performed:

Protocol I-1: This protocol included 1) Rats with LPS induced lung inflammation for 24 hours (n=2) and 2) control rats (n=2).
Protocol I-II: This protocol included 1) Rats with LPS induced lung inflammation for 24 hours which were divided into the following treatment groups: 1) rats were treated with saline at onset of LPS induction and after 12 hours (n=6), 2) rats were treated with α-MSH (Phoenix Pharmaceutical Inc, Mountain View, CA, 50μg, i.v.) at onset of LPS induction and after 12 hours (n=6), 3) rats were treated with Epoitin (100 U/kg/day i.p.) at LPS induction and after 12 hours and 4) rats treated with a combination of epoitin (100 U/kg/day i.p.) and $\alpha$-MSH (50$\mu$g, i.v.) at the onset of LPS and 12 hours later.

Protocol II: Systemic LPS-infusion:

II-1. Controls. Untreated rats without LPS infusion.

II-2 Vehicle: Rats receiving LPS infusion treated with 0.5 ml 0.9 % NaCl twice daily.

II-3 rh-EPO: Rats receiving LPS infusion treated with 200 I.U. epoitin alpha (EPO)/kg body weight in 0.5 ml 0.9% NaCl twice daily.

II-4 $\alpha$-MSH: Rats receiving LPS infusion treated with 200 $\mu$g $\alpha$-melanocyte stimulating hormone ($\alpha$-MSH)/kg body weight in 0.5 ml 0.9% NaCl twice daily.

II-4 $\alpha$-MSH+rh-EPO: Rats receiving LPS infusion treated with 200 $\mu$g $\alpha$-MSH/kg body weightand 200 I.U.EPO/kg body weight in 0.5 ml 0.9% NaCl twice daily.

For protocols II-3, II-4 and II-5 the compounds ($\alpha$-MSH and/or rh-EPO) were given i.v.. The first injections were given 12 hours after the implantation of the osmotic minipump.

Protocol III. Obstruction of the urinary tract

Protocol III-1: This protocol included 1) Rats with BUO for 24 hours (n=20) and 2) sham-operated rats (n=10). The BUO animals were divided into two groups: $\alpha$-MSH nontreated (n=10) and $\alpha$-MSH treated (n=10). $\alpha$-MSH (Phoenix Pharmaceutical Inc, Mountain View, CA, 50$\mu$g, i.v.) was given at the onset of BUO and 12 hours later.

Protocol III-2: This protocol included 1) Rats with BUO for 24 hours and followed by release for 5 hours (n=10) and 2) Sham operated rats (n=5). The rats with BUO-R were divided into two groups: $\alpha$-MSH nontreated (n=5) and $\alpha$-MSH treated (n=5). $\alpha$-MSH (50$\pm$g, i.v.) was given at the onset of BUO, 12 hours later and at the onset of release. Protocol III-3: This protocol included 1) Rats with BUO for 24 hours and followed by release for 48 hours. The rats were divided into two groups: $\alpha$-MSH nontreated (n=10) and $\alpha$-MSH treated (n=13). $\alpha$-MSH (50$\mu$g) was given with micro-osmotic pump via jugular vein at the onset of BUO. 2) Sham operated rats (n=8) treated with vehicle with micro-osmotic pump.

Protocol III-4: This protocol included rats, which had a detailed examination of renal function before onset and after release of BUO. 1) Rats with BUO for 24 hours followed by release for 48 hours (n=10) and 2) sham-operated rats (n=5). The BUO animals were divided into two groups: $\alpha$-MSH nontreated (n=5) and $\alpha$-MSH treated (n=5). $\alpha$-MSH (Phoenix Pharmaceutical Inc, Mountain View, CA, 50$\mu$g, i.v.) was given at the onset of BUO and 12 hours later.

Protocol III-5: This protocol included 1) Rats with UUO for 24 hours (n=11) and 2) sham-operated rats (n=5). The UUO animals were divided into two groups: $\alpha$-MSH nontreated (n=5) and $\alpha$-MSH treated (n=6). $\alpha$-MSH (Phoenix Pharmaceutical Inc, Mountain View, CA, 50$\mu$g, i.v.) was given at the onset of UUO and 12 hours later.

Protocol III-6: This protocol included 1) Rats with BUO for 24 hours and 2) sham-operated rats. The BUO animals were divided into 4 groups: Non-treated rats (n=4), $\alpha$-MSH treated (n=4)($\alpha$-MSH (Phoenix Pharmaceutical Inc, Mountain View, CA, 50$\mu$g, i.v.) was given at the onset of BUO and 12 hours later), Epoitin treated rats (n=4)(100 U/kg/day i.p.) and rats treated with a combination of epoitin (100 U/kg/day i.p.) and $\alpha$-MSH (50$\mu$g, i.v.) was given at the onset of BUO and 12 hours later.

Protocol IV: Experimental nephritic syndrome.

Protocol IV-1. Rats were treated with adriamycin (7.5 mg/kg i.p.; n=12) and followed for 21 days, respectively. Shams received saline i.p. (n=6). Half of the adriamycin treated animals received $\alpha$-MSH in osmotic minipumps (50 ug/day) during the entire experiments. The other half received vehicle (saline) in osmotic minipumps. In another series of experiments $\alpha$-MSH treatment was administered (at the time of adriamycin injection and 6, 24 and 48 hours after adriamycin injection).

Protocol IV-2. Rats were treated with purimycin (100 mg/kg i.p., n=6) and followed for 11days, respectively. Half recieved $\alpha$-MSH treatment (at the time of PAN injection and 6, 24 and 48 hours after PAN injection).

Protocol IV-3. Rats were treated with adriamycin (7.5 mg/kg i.p.; n=8) and followed for 21 days, respectively. Shams received saline i.p. (n=6). Half of the adriamycin treated animals received epoetin (100 units/kg/day) during the entire experiments. The other half received vehicle (saline).

Protocol IV-4. Rats were treated with purimycin aminoglycoside (100 mg/kg i.p., n=6) and followed for 11 days, respectively. Half recieved epoetin (100 units/kg/day).

Protocol IV-5. Rats were treated with purimycin (100 mg/kg i.p., n=6) and followed for 11days, respectively. Half recieved $\alpha$-MSH treatment (at the time of PAN injection and 6, 24 and 48 hours after PAN injection) and in addition they received epoetin (100 units/kg/day).

*Operative procedures:*

*Catheterisation of the Jugular Vein*

The jugular vein was exposed at least 1 cm. The tip of the catheter was inserted into the vein and pushed forwards towards the heart (about 2.5cm) filled with a heparin-saline solution ("heparin lock"). The catheter was then tied into the blood vessel with the ligature. With a small incision made in the dorsal nape of the neck the catheter was passed subcutaneously from the site of the entry of the catheter of the jugular vein to the dorsal incision down the side of the neck to emerge anterior. And a micro-osmotic pump was connected with the end of catheter.

*Permanent bladder catherization*

Catheters were permanently placed in the bladder for urine collection. One week before the experiment, the animals were anesthetized with halothane/$N_2O$. Using aseptic surgical techniques, sterile Tygon™ catheters (Norton Performance Plastics, Arkon, OH) were advanced into the abdominal aorta and the inferior vena cava via the femoral vessels. A sterile chronic suprapubic catheter was implanted into the bladder. After instrumentation, the rats were infused with saline subcutaneously (5 ml) and given a long-acting analgesic, Buprenorphinum (Temgesic™; Reckitt & Colman, Hull, United Kingdom), subcutaneously and housed individually. After a recovery period of 5 to 6 d, the rats were acclimatized to restriction by daily training sessions in restraining cages. The duration of each daily session was gradually increased from 1 to 3 h a day.

*Primary Antibodies*

For semiquantitative immunoblotting, previously characterized mouse monoclonal and affinity purified rabbit polyclonal antibodies were used:

1) AQP2 (LL127 1:6000): An affinity purified polyclonal antibody to AQP2
2) AQP1 (LL266 1:3000): An affinity purified polyclonal antibody to AQP1
3) AQP3 (LL1781:400): An affinity purified polyclonal antibody to AQP3.
4) AQP4 (LL182AP): An affinity purified polyclonal antibody to AQP4 has previously been characterized.
4) NHE-3 (LL546AP): An affinity purified polyclonal antibody to NHE-3 has previously been characterized.
5) NaPi-2 (LL696AP): An affinity purified polyclonal antibody to type II Na-Pi cotransporter (NaPi-2) which was raised against the final 24 amino acids of COOH-terminal sequence has previously been characterized.
6) Na,K-ATPase: A monoclonal antibody against the $\alpha$-1 subunit of Na,K-ATPase has previously been characterized.
7) BSC-1 (LL320AP): An affinity purified polyclonal antibody to the apical Na-K-2Cl cotransporter of the thick ascending limb has previously been characterized.
8) TSC (LL573AP): An affinity purified polyclonal antibody to the apical thiazide-sensitive Na-Cl cotransporter of the distal convoluted tubule has previously been characterized.

*Clearance studies*

Weight, water intake, food intake and urine output were observed during the rats were maintained in the metabolic cages. Urine was collected over 24-h periods throughout the study. Urine volume, osmolality, creatinine, sodium and potassium concentration were measured. Plasma was collected from abdominal aorta at the time of sacrifice for measurement of sodium and potassium concentration, creatinine, and osmolality.
In protocols 4 and 9 detailed examinations of renal function was performed: The experiments were carried out between 8 a.m. and 1 p.m. The rats were transferred to a restraining cage and connected to infusion pumps via the vein catheter and to a BP transducervia the arterial catheter. Urine was collected in three periods of 20 min preceded by an equilibration period of 105 min. Throughout the experiment, a half isotone saline (77 mM NaCl) was infused at a rate of 70 $\mu$l/min to maintain a minimum urine flow necessary for accuracy of the bladder emptying. $^{14}$C-tetraethylammonium bromide (0.83 $\mu$Ci/ml ; New England Nuclear, Boston, MA), together with $^3$H-inulin (2.5 $\mu$Ci/ml; Amersham, Rainham, United Kindgom) and LiCl (13 mmol/L), were infused together with the saline as markers of effective renal plasma flow (ERPF), GFR, and tubular fluid delivery from proximal tubules ($V_{prox}$), respectively. A bolus of markers four times the continuous infusion velocity was given in the first 15 min. Blood samples (200 $\mu$l) were drawn from the arterial catheter after 105 and 165 min. Blood substitution with donor blood was given after each blood sample. Mean arterial BP was recorded continuously using a Uniflow™ transducer (Baxter, Irvine, CA) connected to a preamplifier and PC registration. Clearance experiments were carried out in BUO and SHAM rats 7 days prior to obstruction and 48 hours after release of BUO.

*Analysis*

Urine volume was determined by gravimetric means. Li$^+$ concentration was determined in plasma and urine by flame emission photometry and atomic absorption spectrophotometry, respectively. $^{14}$C-tetraethylammonium (TEA) and $^3$H-inulin in plasma and urine were determined by dual label liquid scintillation counting (Wallac™ model 1409; Helsinki, Finland). Sample (15 μl) and 285 μl of water were mixed with 2.5 ml of scintillation liquid (Ultima Gold™; Packard Instruments, Meriden, CT). Correction of dpm was performed by automatic efficiency control.

*Calculations*

Renal clearances (C) were calculated by the standard formula:

$$C = U \times V/P$$

where U is urine concentration, V is urine flow rate, and Pis plasma concentration.

In previous studies, the renal extraction fraction of TEA has been shown to approximate 90%, and the validity of TEA as an estimate of ERPF has previously been documented. By use of $C_{TEA}$, $C_{IN}$, and $C_{LI}$, the following parameters were calculated:

ERPF = Effective renal plasma flow (CTEA)
GFR = Glomerular filtration rate (Cin)

*Membrane fractionation for immunoblotting*

Inner medulla and whole kidneys were homogenized (0.3 M sucrose, 25 mM imidazole, 1 mM EDTA, pH 7.2, containing 8.5 μM leupeptin. 1 mM phenylmethyl sulfonylfluoride) using an ultra-turrax T8 homogenizer (IKA Labortechnik, Germany), at 5 strokes for 20 seconds (inner medulla) or at 5.5 strokes for 30 seconds (whole kidney) and the homogenate was centrifuged in an Eppendorf centrifuge at 4000 g for 15 minutes at 4°C to remove whole cells, nuclei and mitochondria. Gel samples (Laemmli sample buffer containing 2% SDS) were made of this pellet.

*Electrophoresis and immunoblotting*

Samples of membrane fractions from inner medulla and whole kidney were run on 12% or 8-16% gradient polyacrylamide minigels (Bio-Rad Mini Protean II) for AQP1, AQP2 and AQP3 (or other renal transporters). For each gel, identical gel was run in parallel and subjected to Coomassie staining to assure identical loading. Then gels were subjected to immunoblotting. After transfer by electroelution to nitrocellulose membranes, blots were blocked with 5% milk in PBS-T (80 mM Na$_2$HPO$_4$, 20 mM NaH$_2$PO$_4$, 100 mM NaCl, 0.1% Tween 20, pH 7.5) for 1 h and incubated overnight at 4°C with affinity-purified primary antibodies (see above). The labeling was visualized with horseradish peroxidase (HRP)-conjugated secondary antibodies (P448, DAKO, Glostrup Denmark, diluted as 1:3000) using an enhanced chemiluminescence system (ECL, Amersham International, UK).

*Quantitation of total kidney levels of AQPs and other renal transporters.*

ECL films with bands within the linear range were scanned using an AGFA scanner (ARCUS II) and Corel Photopaint Software to control the scanner. For AQP1 and AQP2, both the 29-kDa and the 35- to 50-kDa bands (corresponding to nonglycosylated and the glycosylated species) were scanned. For AQP3, both the 27-kDa and the 33- to 40-kDa bands (corresponding to nonglycosylated and the glycosylated species) were scanned. The labeling density was determined of blots where samples of kidneys from α-MSH treated and nontreated groups were run together with samples from control kidneys. The labeling density was corrected by densitometry of coomassie stained gels. (i.e. to control for minor difference in loading)

*Quantitation of urinary protein excretion*

To determine the levels of proteinuria in experimental nephrotic syndrome urine was examined daily using Albym (Boehringer-Mannheim).
Statistical analyses Values were presented in the text as means $\pm$ standard errors. Comparisons between groups were made by unpaired t-test. P values < 0.05 were considered significant.

Results

*LPS inhalation for 20 min is associated with a marked inflammatory response in the lung tissue*

LPS induced a dramatic influx of inflammatory cells in the lung tissue. Differential counting revealed that lung tissue exposed for LPS had large numbers of neutrophil leucocytes (68.5±8%), eosinofiles (8.6±4 %), lymphocytes (15.3 ±6%), and monocytes (8±2%) (Table 1).

*α-MSH prevents influx of inflammatory cells in lung tissue exposed with LPS*

Treatment with α-MSH reduced the influx of inflammatory cells in the lung tissue exposed with LPS. Differential counting revealed that the number of eosinofils were reduced (1.8±1.3% vs. 3.6±2.7%), and neutrophil granulocytes were markedly reduced (10.6±10.6% vs. 34.4±26.1%) (Table 1).

*Epoitin prevents influx of inflammatory cells in lung tissue exposed with LPS*

Treatment with epoitin reduced the influx of inflammatory cells in the lung tissue exposed with LPS. Differential counting revealed that the number of eosinofils were reduced (0.7±0.6% vs. 3.6±2.7%), and neutrophil granulocytes were markedly reduced (10.9±12.7% vs. 34.4±26.1%) (Table 1).

*Combined treatment with α-MSH and epoitin almost completely prevented the influx of inflammatory cells in lung tissue exposed with LPS*

Combined treatment with α-MSH and epoitin dramatically reduced the influx of inflammatory cells in the lung tissue exposed with LPS. Differential counting revealed that the number of eosinofiles were reduced (0.6±0.51% vs. 3.6±2.7%), and neutrophile granulocytes were markedly reduced (3.8±1.9% vs. 34.4±26.1%) (Table 1). In addition the influx of monocytes was also significantly prevented with this treatment (6.0±4.1 % vs. 13.9±8.4%) (Table 1).

*LPS administration in osmotic minipumps for 3 days is associated with a severe inflammatory response and severe renal failure*

The rats were followed for three days after the implantation of the osmotic minipump with LPS. Glomerular filtration rate as evaluated through creatinine clearance was significantly reduced with 68% in the LPS-Vehicle group compared to the untreated control rats. Thus the animals had severe renal failure. The mortality was 20%.

*Treatment with α-MSH or with epoitin or with combined α-MSH and epoitin prevented the inflammatory-induced renal failure seen in response to LPS administration*

During 3 days of LPS-administration the mortality in the vehicle treated group were 20% (1 out of 5). All animals survived in the other groups, i.e. rh-EPO treatment, α-MSH treatment; combined treatment with low dose rh-EPO and α-MSH, and in the sham-operated controls. The parameters of renal function and other parameters are described in Table 2. Glomerular filtration rate as evaluated through creatinin clearance was significantly reduced with 68% in the LPS-Vehicle group compared to the untreated control rats. Both rh-EPO and α-MSH treatment increased GFR in the LPS treated rats by 51% and 57%, respectively. However GFR was still severely affected compared to the untreated control rats. The combined treatment with rh-EPO and α-MSH significantly increased GFR by 111 % compared to the LPS-Vehicle group suggesting a synergestic effect of the combined treatment with rh-EPO and α-MSH.

*BUO for 24 hours, 5 and 48 hours after release of BUO are associated with reduced AQP1, AQP2 and AQP3*

As previously studied, immunoblotting revealed that 24 h of BUO and 48 hours after release of BUO were associated with a significant downregulation of AQP-2 expression compared with sham-operated controls (13±4%). Semiquantitative immunoblotting from the inner medulla of rats with 24h BUO and BUO-R for, 5 and 48 hours revealed that AQP3 expression was persistently downregulated. Both AQP3 bands (the 27-kDa and 33-to 40-kDa bands) were decreased proportionately. Densitometric analysis revealed a significant decrease in AQP3 expression in rats with 24 h BUO to 19±4% of sham levels (100±7%, p<0.05). Furthermore, AQP3 protein levels were marked decreased at 5 hours after release to 15±5% of sham levels (100±9%, p<0.05) and at 48 hours after release to 10±5% of sham level (100±1 %, p<0.05).

Semiquantitative immunoblotting using membrane fractions prepared from the whole kidney of rats with 24h BUO and BUO-R for 5 and 48 hours revealed that AQP1 expression was persistently downregulated. Both AQP1 bands (the 29-kDa and 35- to 50-kDa bands) were decreased proportionately. Densitometric analysis revealed a significant decrease in AQP1 expression in rats with 24 h BUO to 53±7% of sham levels (100±9%, p<0.05). Furthermore, AQP1 protein levels remained markedly decreased at 5 hours after release of BUO to 7 ±2% of sham levels (100±22%, p<0.05) and at 48 hours after release to 30±5% of sham level (100±10%, p<0.05).

*BUO for 24 hours, 5 and 48 hours after release of BUO are associated with downregulation of Na, K-ATPase*

Semiquantitative immunoblotting using membrane fraction prepared from whole kidney of rats with 24 h BUO, and BUO followed by 5 h and 48 h after release of BUO showed a persistant downregulation of Na,K-ATPase to 35 - 50% of control levels.

*α-MSH partially prevents AQP2 and AQP3 downregulation in response to 24 h of BUO*

Semiquantitative immunoblotting using membrane fractions prepared from the inner medulla of rats with 24 h BUO and sham operated control rats revealed that α-MSH treatment significantly increased AQP2 expression were compared with nontreated rats (38±5% vs. 13±4%, p<0.05). At the same time, immunoblotting also showed that expression of AQP3 in rats with 24 h BUO was significantly upregulated in response to α-MSH treatment compared with nontreated rats (44±3% vs. 19±4%, p<0.05).

Plasma concentrations data showed that in α-MSH treated rats with 24 h BUO plasma sodium levels had a marked increase compared with 24 h BUO rats without α-MSH treatment from 135±2mmol/L to 139±0.6 mmol/L. There was no difference between α-MSH treated rats and sham operated rats.

*α-MSH prevents AQP3 and AQP1 downregulation in response to 24 h BUO followed by release for 5 hours*

Semiquantitative immunoblotting using membrane fractions prepared from the inner medulla of 24 h BUO rats followed by release for 5 hours and sham operated rats revealed that with α-MSH treatment AQP3 expression was significantly increased compared with nontreated rats with 24 h BUO followed by release for 5 hours form 14±5% to 34±4% of sham level(100±14%).

Semiquantitative immunoblotting using membrane fractions prepared from whole kidney of rats with 24 h BUO followed by release for 5 hours and sham operated control rats revealed that with α-MSH treatment significantly increased AQP1 expression levels compared with nontreated BUO-R rats (81±21% vs. 7±2%, p<0.05) (Fig 2 A and B). Furthermore, immunoblotting prepared from outer medulla and cortex also showed the same result that in α-MSH treated rats AQP1 expression was a marked increase significantly compared with nontreated rats from 35±2% to 62±9% of sham level.

*α-MSH prevents AQP1 downregulation in response to 24 h BUO followed by release for 48 hours*

In rats with 24 h BUO and 5 hours after release of BUO, α-MSH was given every 12 hours. In rats with 48 hours after release of BUO, α-MSH was given with micro-osmotic pump continuously. Semiquantitative immunoblotting using membrane fractions prepared from the whole kidney of 24 h BUO rats followed by release for 48 hours and sham operated rats revealed that with α-MSH treatment the levels of AQP1 expression were significantly increased compared with nontreated rats with 48 hours after release of BUO form 24±5% to 58±6% of sham level (100±10%). Furthermore, immunoblotting prepared from outer medulla and cortex also showed the same result that in α-MSH treated rats AQP1 expression was a marked increase compared with nontreated rats to 73±8% of sham level.

*α-MSH completely normalise expression of Na, K-ATPase during BUO and 5 and 48 hours after release of BUO*

Semiquantitative immunoblotting using membrane fractions prepared from whole kidney of rats with 24 h BUO showed that Na,K-ATPase levels were completely normalized. Also, 24 h BUO followed by 5 h of release and sham operated control rats revealed that α-MSH completely normalized Na-K-ATPase levels (102±14%). α-MSH treatment also completely normalized Na-K-ATPase levels 48 hours after release of BUO (114±10%).

*Treatment with α-MSH prevents the increase in plasma creatinine and restores GFR and RPF in response to 24 hours of BUO followed by 48 hours of release*

Plasma creatinine is an important marker of glomerular filtration rate. In rats with BUO for 24 h followed by 48 of release and sham operated control rats α-MSH treatment significantly prevented the dramatic increase in plasma creatinine (indicating severe renal insufficiency). Plasma creatinine levels were significantly reduced in α-MSH treated rats (79±34 μmol/l vs. 160±34 μmol/l, p<0.05,). In addition, plasma urea which is another important marker of renal function almost normalized in rats treated with α-MSH (13±2 mmol/l vs. 35±9 mmol/l, p<0.05). Also GFR and ERPF were completely normalized (Fig 3 A and B).

*Epoitin partially prevents AQP2 downregulation in response to 24 h of BUO*

Semiquantitative immunoblotting using membrane fractions prepared from the inner medulla of rats with 24 h BUO and sham operated control rats revealed that epoitin treatment significantly increased AQP2 expression were compared with nontreated rats (30±18% vs. 16±3%, p<0.05).

*Preventive effect of α-MSH, or Epoetin treatment on adriamycin and purimycin-induced nephrotic syndrome in rats.*

Treatment with α-MSH for 3-4 days after i.p injection of adriamycin (Protocol IV-1) significantly prevented the ascites production by 89 ± 7% indicating a marked preventive effect of α-MSH on experimentally induced nephrotic syndrome. A similar dramatic effect was also observed in low dose purimycin treatment (80±8% reduction, n=6) and a marked reduction in proteinuria (Protocol IV-2). A less potent effect was seen in very servere purimycin (high dose) induced nephrotic syndrome. EPO treatment once every 24 hours also prevented the ascites formation in adriamycin-induced nephrotic syndrome and co-treatment of epoetin and α-MSH produced an even greater effect.

TABLES

Table 1.

A) Differential counting showed that LPS induced a marked inflammatory response in the lungs

B) Differential counting showed marked reduction in the number of inflammatory cells in the lungs with all three treatment regimens. Importantly the data show a dramatic reduction in response to combined treatment with α-MSH+Epoitin.

| A | Differential counting of cells % | | | |
|---|---|---|---|---|
| Treatment | Eosinofiles | Neutrofiles | Lymfocytes | Monocytes |
| Controls | 0 | 0 | 0 | 0 |
| LPS | 8.6±4 | 68.5±8 | 15.2±6 | 8±2 |

| B | Differential counting of cells % | | | |
|---|---|---|---|---|
| Treatment | Eosinofiles | Neutrofiles | Lymfocytes | Monocytes |
| Vehicle | 3.6±2.7 | 34.4±26.1 | 48.5±20.9 | 13.9±8.4 |
| α-MSH | 1.8±1.3 | 10.6±10.6 | 72.3±12.1 | 12.3±7.4 |
| Epoitin | 0.7±0.6 | 10.9±12.7 | 77.1±9.9 | 11.4±4.6 |
| α-MSH+Epoitin | 0.6±0.51 | 3.8±1.9 | 89.4±4.1 | 6.0±4.1 |

Table 2. Effects of vehicle (0.9% NaCl); rh-EPO (200 U/kg b.w.); α-MSH (200 μg/kg b.w.) and the combination of rh-EPO (200 U/kg b.w.) and α-MSH (200 μg/kg b.w.) on renal function in rats treated with continious LPS infusion (200 μg/kg/h) during three days.

| | Creatinin Clearance ml/min |
|---|---|
| **Control:** | 1.11 ± 0.08 |
| **Vehicle:** | 0.35 ± 0.07 |
| **rh-EPO:** | 0.53 ± 0.06 * |
| **α-MSH:** | 0.55 ± 0.05 * |

(continued)

|  | Creatinin Clearance ml/min |
|---|---|
| **α-MSH+rh-EPO:** | 0.74 ± 0.06 * # □: |

*: different from Vehicle treated rats; p<0.05; #: different from rh-EPO treated rats; p<0.05;
□: different from α-MSH treated rats; p<0.05.

Reference List

1. Apostol, E., C. A. Ecelbarger, J. Terris, A. D. Bradford, P. Andrews, and M. A. Knepper. Reduced renal medullary water channel expression in puromycin aminonucleoside-induced nephrotic syndrome. J.Am.Soc.Nephrol. 8: 15-24, 1997.

2. Berens, K. L., R. R. Verani, and D. R. Luke. Role of neutrophils and macrophages in experimental nephrosis of the rat. Ren Fail. 20: 53-63, 1998.

3. Bernaudin, M., H. H. Marti, S. Roussel, D. Divoux, A. Nouvelot, E. T. MacKenzie, and E. Petit. A potential role for erythropoietin in focal permanent cerebral ischemia in mice. J.Cereb.Blood Flow Metab 19: 643-651, 1999.

4. Bhardwaj, R. and J. Blanchard. Controlled-release delivery system for the alpha-MSH analog melanotan-I using poloxamer 407. J.Pharm.Sci. 85: 915-919, 1996.

5. Bohle, A., S. Mackensen-Haen, and H. von Gise. Significance of tubulointerstitial changes in the renal cortex for the excretory function and concentration ability of the kidney: a morphometric contribution. Am.J.Nephrol. 7: 421-433, 1987.

6. Borghi, L., T. Meschi, F. Amato, A. Novarini, A. Giannini, C. Quarantelli, and F. Mineo. Nifedipine and methylprednisolone in facilitating ureteral stone passage: a randomized, double-blind, placebo-controlled study [see comments]. J.Urol. 152: 1095-1098, 1994.

7. Bunn, H. F., J. Gu, L. E. Huang, J. W. Park, and H. Zhu. Erythropoietin: a model system for studying oxygen-dependent gene regulation. J.Exp.Biol. 201 (Pt 8): 1197-1201, 1998.

8. Canbolat, O., J. Fandrey, and W. Jelkmann. Effects of modulators of the production and degradation of hydrogen peroxide on erythropoietin synthesis. Respir.Physiol 114: 175-183, 1998.

9. Catania, A., N. Rajora, F. Capsoni, F. Minonzio, R. A. Star, and J. M. Lipton. The neuropeptide alpha-MSH has specific receptors on neutrophils and reduces chemotaxis in vitro. Peptides 17: 675-679, 1996.

10. Chhajlani, V. and J. E. Wikberg. Molecular cloning and expression of the human melanocyte stimulating hormone receptor cDNA. FEBS Lett. 309: 417-420, 1992.

11. Chhajlani, V., R. Muceniece, and J. E. Wikberg. Molecular cloning of a novel human melanocortin receptor [published erratum appears in Biochem Biophys Res Commun 1996 Jan 17;218(2):638]. Biochem.Biophys.Res.Commun. 195: 866-873, 1993.

12. Chye, R. and N. Lickiss. The use of corticosteroids in the management of bilateral malignant ureteric obstruction. J.Pain Symptom.Manage. 9: 537-540, 1994.

13. Diamond, J. R., D. Kees-Folts, G. Ding, J. E. Frye, and N. C. Restrepo. Macrophages, monocyte chemoattractant peptide-1, and TGF-beta 1 in experimental hydronephrosis. Am.J.Physiol 266: F926-F933, 1994.

14. Digicaylioglu, M., S. Bichet, H. H. Marti, R. H. Wenger, L. A. Rivas, C. Bauer, and M. Gassmann. Localization of specific erythropoietin binding sites in defined areas of the mouse brain. Proc.Natl.Acad.Sci.U.S.A 92: 3717-3720, 1995.

15. Ehleben, W., T. Porwol, J. Fandrey, W. Kummer, and H. Acker. Cobalt and desferrioxamine reveal crucial members of the oxygen sensing pathway in HepG2 cells. Kidney Int. 51: 483-491, 1997.

16. el Nahas, A. M. Pathways to renal fibrosis. Exp.Nephrol. 3: 71-75, 1995.

17. Fandrey, J. and H. F. Bunn. In vivo and in vitro regulation of erythropoietin mRNA: measurement by competitive polymerase chain reaction. Blood 81: 617-623, 1993.

18. Fandrey, J., S. Frede, and W. Jelkmann. Role of hydrogen peroxide in hypoxia-induced erythropoietin production. Biochem.J. 303 (Pt 2): 507-510, 1994.

19. Fandrey, J., S. Frede, W. Ehleben, T. Porwol, H. Acker, and W. Jelkmann. Cobalt chloride and desferrioxamine antagonize the inhibition of erythropoietin production by reactive oxygen species. Kidney Int 51: 492-496, 1997.

20. Fernandez-Llama, P., P. Andrews, S. Nielsen, C. A. Ecelbarger, and M. A. Knepper. Impaired aquaporin and urea transporter expression in rats with adriamycin-induced nephrotic syndrome. Kidney Int. 53 : 1244-1253, 1998.

21. Fernandez-Llama, P., P. Andrews, R. Turner, S. Saggi, J. Dimari, T. H. Kwon, S. Nielsen, R. Safirstein, and M. A. Knepper. Decreased abundance of collecting duct aquaporins in post-ischemic renal failure in rats. J.Am.Soc.Nephrol. 10: 1658-1668, 1999.

22. Frokiaer, J., A. S. Nielsen, L. Knudsen, J. C. Djurhuus, and E. B. Pedersen. The effect of indomethacin infusion on renal hemodynamics and on the renin-angiotensin system during unilateral ureteral obstruction of the pig. J.Urol. 150: 1557-1563, 1993.

23. Goldberg, M. A., G. A. Glass, J. M. Cunningham, and H. F. Bunn. The regulated expression of erythropoietin by two human hepatoma cell lines. Proc. Natl.Acad.Sci. U. S.A 84: 7972-7976, 1987.

24. Goldberg, M. A., S. P. Dunning, and H. F. Bunn. Regulation of the erythropoietin gene: evidence that the oxygen sensor is a heme protein. Science 242: 1412-1415, 1988.

25. Goldberg, M. A., C. C. Gaut, and H. F. Bunn. Erythropoietin mRNA levels are governed by both the rate of gene transcription and posttranscriptional events. Blood 77: 271-277, 1991.

26. Gorlach, A., J. Fandrey, G. Holtermann, and H. Acker. Effects of cobalt on haem proteins of erythropoietin-producing HepG2 cells in multicellular spheroid culture. FEBS Lett. 348: 216-218, 1994.

27. Hartmeyer, M., T. Scholzen, E. Becher, R. S. Bhardwaj, T. Schwarz, and T. A. Luger. Human dermal microvascular endothelial cells express the melanocortin receptor type 1 and produce increased levels of IL-8 upon stimulation with alpha-melanocyte-stimulating hormone. J.Immunol. 159: 1930-1937, 1997.

28. Ishibashi, K., S. Sasaki, K. Fushimi, T. Yamamoto, M. Kuwahara, and F. Marumo. Immunolocalization and effect of dehydration on AQP3, a basolateral water channel of kidney collecting ducts. Am.J.Physiol 272: F235-F241, 1997.

29. Ishidoya, S., J. Morrissey, R. McCracken, A. Reyes, and S. Klahr. Angiotensin II receptor antagonist ameliorates renal tubulointerstitial fibrosis caused by unilateral ureteral obstruction. Kidney Int. 47: 1285-1294, 1995.

30. Jaenike, J. R. The renal functional defect of postobstructive nephyropathy. The effects of bilateral ureteral obstruction in the rat. J.Clin.Invest 51: 2999-3006, 1972.

31. Jelkmann, W. Erythropoietin: structure, control of production, and function. Physiol Rev. 72: 449-489, 1992.

32. Jones, E. A., A. Shahed, and D. A. Shoskes. Modulation of apoptotic and inflammatory genes by bioflavonoids and angiotensin II inhibition in ureteral obstruction [In Process Citation]. Urology 56: 346-351, 2000.

33. Jones, E. A. and D. A. Shoskes. The effect of mycophenolate mofetil and polyphenolic bioflavonoids on renal ischemia reperfusion injury and repair. J.Urol. 163: 999-1004, 2000.

34. Kaneto, H., J. Morrissey, and S. Klahr. Increased expression of TGF-beta 1 mRNA in the obstructed kidney of rats with unilateral ureteral ligation. Kidney Int. 44: 313-321, 1993.

35. Kaneto, H., J. Morrissey, R. McCracken, A. Reyes, and S. Klahr. Enalapril reduces collagen type IV synthesis and expansion of the interstitium in the obstructed rat kidney. Kidney Int. 45: 1637-1647, 1994.

36. Klahr, S. and J. J. Morrissey. The role of growth factors, cytokines, and vasoactive compounds in obstructive nephropathy. Semin.Nephrol. 18: 622-632, 1998.

37. Klahr, S. and J. Morrissey. Angiotensin II and gene expression in the kidney. Am.J.Kidney Dis. 31: 171-176, 1998.

38. Kramer, B. K, M. Bucher, P. Sandner, K. P. Ittner, G. A. Riegger, T. Ritthaler, and A. Kurtz. Effects of hypoxia on growth factor expression in the rat kidney in vivo. Kidney Int. 51: 444-447, 1997.

39. Kuncio, G. S., E. G. Neilson, and T. Haverty. Mechanisms of tubulointerstitial fibrosis. Kidney Int. 39: 550-556, 1991.

40. Kwon, T. H., J. Frokiaer, P. Fernandez-Llama, M. A. Knepper, and S. Nielsen. Reduced abundance of aquaporins in rats with bilateral ischemia-induced acute renal failure: prevention by alpha-MSH. Am.J.Physiol 277: F413-F427, 1999.

41. Lipton, J. M. and A. Catania. Anti-inflammatory actions of the neuroimmunomodulator alpha-MSH. Immunol. Today 18: 140-145, 1997.

42. Luger, T. A., T. Scholzen, and S. Grabbe. The role of alpha-melanocyte-stimulating hormone in cutaneous biology. J.Investig.Dermatol.Symp.Proc. 2: 87-93, 1997.

43. Marti, H. H., R. H. Wenger, L. A. Rivas, U. Straumann, M. Digicaylioglu, V. Henn, Y. Yonekawa, C. Bauer, and M. Gassmann. Erythropoietin gene expression in human, monkey and murine brain. Eur.J.Neurosci. 8: 666-676, 1996.

44. Morishita, E., S. Masuda, M. Nagao, Y. Yasuda, and R. Sasaki. Erythropoietin receptor is expressed in rat hippocampal and cerebral cortical neurons, and erythropoietin prevents in vitro glutamate- induced neuronal death. Neuroscience 76: 105-116, 1997.

45. Moriyama, T., N. Kawada, A. Ando, A. Yamauchi, M. Horio, K. Nagata, E. Imai, and M. Hori. Up-regulation of HSP47 in the mouse kidneys with unilateral ureteral obstruction. Kidney Int. 54: 110-119, 1998.

46. Morrissey, J. J. and S. Klahr. Rapid communication. Enalapril decreases nuclear factor kappa B activation in the kidney with ureteral obstruction. Kidney Int. 52: 926-933, 1997.

47. Morrissey, J. J. and S. Klahr. Differential effects of ACE and AT1 receptor inhibition on chemoattractant and adhesion molecule synthesis. Am.J.Physiol 274: F580-F586, 1998.

48. Morrissey, J. J. and S. Klahr. Effect of AT2 receptor blockade on the pathogenesis of renal fibrosis. Am.J.Physiol 276: F39-F45, 1999.

49. Mountjoy, K. G., L. S. Robbins, M. T. Mortrud, and R. D. Cone. The cloning of a family of genes that encode the melanocortin receptors. Science 257: 1248-1251, 1992.

50. Nagle, R. B., M. R. Kneiser, R. E. Bulger, and E. P. Benditt. Induction of smooth muscle characteristics in renal interstitial fibroblasts during obstructive nephropathy. Lab Invest 29: 422-427, 1973.

51. Nielsen, S., S. R. DiGiovanni, E. I. Christensen, M. A. Knepper, and H. W. Harris. Cellular and subcellular immunolocalization of vasopressin-regulated water channel in rat kidney. Proc.Natl.Acad.Sci.U.S.A 90: 11663-11667, 1993.

52. Nielsen, S., C. L. Chou, D. Marples, E. I. Christensen, B. K. Kishore, and M. A. Knepper. Vasopressin increases water permeability of kidney collecting duct by inducing translocation of aquaporin-CD water channels to plasma membrane. Proc.Natl.Acad.Sci.U.S.A 92: 1013-1017, 1995.

53. Perlmutter, A., L. Miller, L. A. Trimble, D. N. Marion, E. D. Vaughan, Jr., and D. Felsen. Toradol, an NSAID used for renal colic, decreases renal perfusion and ureteral pressure in a canine model of unilateral ureteral obstruction. J.Urol. 149: 926-930, 1993.

54. Petersen, J. S., M. Shalmi, H. R. Lam, and S. Christensen. Renal response to furosemide in conscious rats: effects of acute instrumentation and peripheral sympathectomy. J.Pharmacol.Exp.Ther. 258: 1-7, 1991.

55. Porwol, T., W. Ehleben, K Zierold, J. Fandrey, and H. Acker. The influence of nickel and cobalt on putative members of the oxygen- sensing pathway of erythropoietin-producing HepG2 cells. Eur.J.Biochem. 256: 16-23, 1998.

56. Rajora, N., G. Boccoli, D. Burns, S. Sharma, A P. Catania, and J. M. Lipton. alpha-MSH modulates local and circulating tumor necrosis factor-alpha in experimental brain inflammation. J.Neurosci. 17: 2181-2186, 1997.

57. Rajora, N., G. Boccoli, A. Catania, and J. M. Lipton. alpha-MSH modulates experimental inflammatory bowel disease. Peptides 18: 381-385, 1997.

58. Ratcliffe, P. J., P. H. Maxwell, and C. W. Pugh. Beyond erythropoietin: the oxygen sensor. Nephrol.Dial.Transplant. 12: 1842-1848, 1997.

59. Ratcliffe, P. J., B. L. Ebert, J. D. Firth, J. M. Gleadle, P. H. Maxwell, M. Nagao, J. F. O'Rourke, C. W. Pugh, and S. M. Wood. Oxygen regulated gene expression: erythropoietin as a model system. Kidney Int. 51: 514-526, 1997.

60. Ricardo, S. D., M. E. Levinson, M. R. DeJoseph, and J. R. Diamond. Expression of adhesion molecules in rat renal cortex during experimental hydronephrosis. Kidney Int. 50: 2002-2010, 1996.

61. Sabolic, I., T. Katsura, J. M. Verbavatz, and D. Brown. The AQP2 water channel: effect of vasopressin treatment, microtubule disruption, and distribution in neonatal rats [published erratum appears in J Membr Biol 1995 May;145 (1):107-8]. J.Membr.Biol. 143: 165-175, 1995.

62. Sadamoto, Y., K. Igase, M. Sakanaka, K. Sato, H. Otsuka, S. Sakaki, S. Masuda, and R. Sasaki. Erythropoietin prevents place navigation disability and cortical infarction in rats with permanent occlusion of the middle cerebral artery. Biochem.Biophys.Res.Commun. 253: 26-32, 1998.

63. Sakanaka, M., T. C. Wen, S. Matsuda, S. Masuda, E. Morishita, M. Nagao, and R. Sasaki. In vivo evidence that erythropoietin protects neurons from ischemic damage. Proc.Natl.Acad.Sci.U.S.A 95: 4635-4640, 1998.

64. Salceda, S. and J. Caro. Hypoxia-inducible factor 1alpha (HIF-1alpha) protein is rapidly degraded by the ubiquitin-proteasome system under normoxic conditions. Its stabilization by hypoxia depends on redox-induced changes. J.Biol.Chem. 272: 22642-22647, 1997.

65. Schioth, H. B., V. Chhajlani, R. Muceniece, V. Klusa, and J. E. Wikberg. Major pharmacological distinction of the ACTH receptor from other melanocortin receptors. Life Sci. 59: 797-801, 1996.

66. Schuster, S. J., E. V. Badiavas, P. Costa-Giomi, R. Weinmann, A. J. Erslev, and J. Caro. Stimulation of erythropoietin gene transcription during hypoxia and cobalt exposure. Blood 73: 13-16, 1989.

67. Semenza, G. L. and G. L. Wang. A nuclear factor induced by hypoxia via de novo protein synthesis binds to the human erythropoietin gene enhancer at a site required for transcriptional activation. Mol.Cell Biol. 12: 5447-5454, 1992.

68. Sharma, A. K., S. M. Mauer, Y. Kim, and A. F. Michael. Interstitial fibrosis in obstructive nephropathy. Kidney Int. 44: 774-788, 1993.

69. Sonnenberg, H. and D. R. Wilson. The role of the medullary collecting ducts in postobstructive diuresis. J.Clin.Invest 57: 1564-1574, 1976.

70. Squadrito, F., S. Guarini, D. Altavilla, G. Squadrito, G. M. Campo, M. Arlotta, C. Quartarone, A. Saitta, D. Cucinotta, C. Bazzani, M. M. Cainazzo, C. Mioni, A. Bertolini, and A. P. Caputi. Adrenocorticotropin reverses vascular dysfunction and protects against splanchnic artery occlusion shock. Br.J.Pharmacol. 128 : 816-822, 1999.

71. Squadrito, F., D. Altavilla, G. Squadrito, G. M. Campo, M. Arlotta, C. Quartarone, A. Saitta, and A. P. Caputi. Recombinant human erythropoietin inhibits iNOS activity and reverts vascular dysfunction in splanchnic artery occlusion shock. Br.J.Pharmacol. 127: 482-488, 1999.

72. Star, R. A., N. Rajora, J. Huang, R. C. Stock, A. Catania, and J. M. Lipton. Evidence of autocrine modulation of macrophage nitric oxide synthase by alpha-melanocyte-stimulating hormone. Proc.Natl.Acad.Sci.U.S.A 92: 8016-8020, 1995.

73. Strutz, F. and E. G. Neilson. The role of lymphocytes in the progression of interstitial disease. Kidney Int.Suppl 45: S106-S110, 1994.

74. Strutz, F. Novel aspects of renal fibrogenesis. Nephrol.Dial.Transplant. 10: 1526-1532, 1995.

75. Strutz, F. and G. A. Muller. On the progression of chronic renal disease. Nephron 69: 371-379, 1995.

76. Tan, C. C., K U. Eckardt, J. D. Firth, and P. J. Ratcliffe. Feedback modulation of renal and hepatic erythropoietin mRNA in response to graded anemia and hypoxia. Am.J.Physiol 263: F474-F481, 1992.

77. Van De Merwe, J. P. and H. J. Arendsen. Interstitial cystitis: a review of immunological aspects of the aetiology and pathogenesis, with a hypothesis. BJU.Int. 85: 995-999, 2000.

78. Vanetti, M., C. Schonrock, W. Meyerhof, and V. Hollt. Molecular cloning of a bovine MSH receptor which is highly expressed in the testis. FEBS Lett. 348: 268-272, 1994.

79. Wang, G. L. and G. L. Semenza. General involvement of hypoxia-inducible factor 1 in transcriptional response to hypoxia. Proc.Natl.Acad.Sci.U.S.A 90: 4304-4308, 1993.

80. Wang, G. L. and G. L. Semenza. Desferrioxamine induces erythropoietin gene expression and hypoxia- inducible factor 1 DNA-binding activity: implications for models of hypoxia signal transduction. Blood 82: 3610-3615, 1993.

81. Wang, G. L., B. H. Jiang, and G. L. Semenza. Effect of altered redox states on expression and DNA-binding activity of hypoxia-inducible factor 1. Biochem.Biophys.Res.Commun. 212: 550-556, 1995.

82. Wenger, R. H., I. Kvietikova, A. Rolfs, M. Gassmann, and H. H. Marti. Hypoxia-inducible factor-1 alpha is regulated at the post-mRNA level. Kidney Int. 51: 560-563, 1997.

83. Wong, K. Y., N. Rajora, G. Boccoli, A. Catania, and J. M. Lipton. A potential mechanism of local anti-inflammatory action of alpha- melanocyte-stimulating hormone within the brain: modulation of tumor necrosis factor-alpha production by human astrocytic cells. Neuroimmunomodulation. 4: 37-41, 1997.

84. Xia, Y., J. E. Wikberg, and V. Chhajlani. Expression of melanocortin 1 receptor in periaqueductal gray matter. Neuroreport 6: 2193-2196, 1995.

85. Zanjani, E. D., J. Poster, H. Burlington, L. I. Mann, and L. R. Wasserman. Liver as the primary site of erythropoietin formation in the fetus. J.Lab Clin.Med. 89: 640-644, 1977.

**Claims**

1. Use of EPO optionally in combination with α-MSH or an α-MSH equivalent including the sequence His-Phe-Arg and acting on a melanocortin type 1 receptor for the preparation of a medicament for treatment or prevention of an acute inflammatory condition or disease under non-ischemic conditions in airways and lungs, or in the kidney or in the urinary tract.

**2.** Use according to claim 1 of EPO for the preparation of a medicament for treatment or prevention of an acute inflammatory condition or disease under non-ischemic conditions in airways and lungs, or in the kidney or in the urinary tract.

**3.** Use according to claim 1 of EPO in combination with $\alpha$-MSH for the preparation of a medicament for treatment or prevention of an acute inflammatory condition or disease under non-ischemic conditions in airways and lungs, or In the kidney or in the urinary tract.

**4.** Use according to claim 1 of EPO in combination with an $\alpha$-MSH equivalent including the sequence His-Phe-Arg and acting on a melanocortin type 1 receptor for the preparation of a medicament for treatment or prevention of an acute inflammatory condition or disease under non-ischemic conditions in airways and lungs, or in the kidney or in the urinary tract.

**5.** Use according to any of claims 1-4, wherein the inflammatory condition is caused by an infection.

**6.** Use according to any of claims 1-5, wherein the acute inflammatory condition is caused by a side-effect of drugs or poisoning.

**7.** Use according to any of claims 1-5, wherein the acute inflammatory condition in airways and lungs is an exacerbation of chronic obstructive pulmonary disease (COPD).

**8.** Use according to any of claims 1-5, wherein the acute Inflammatory condition is astma.

**9.** Use according to any of claims 1-5, wherein the acute inflammatory condition in the kidney or in the urinary tract is obstruction of the urinary tract or cystitis.

**10.** Use according to any of the preceding claims, wherein the medicament is to be administered prophylactically for preventing the establishment or progress of the condition, or of any symptom of the condition.

**11.** Use according to any of claims 3-10, wherein EPO and $\alpha$-MSH or an $\alpha$-MSH equivalent including the sequence His-Phe-Arg and acting on a melanocortin type 1 receptor are to be administered independently of each other.

**12.** Use according to any of claims 3-10, wherein EPO and $\alpha$-MSH or an $\alpha$-MSH equivalent including the sequence His-Phe-Arg and acting on a melanocortin type 1 receptor are to be co-administered.

**13.** A pharmaceutical composition comprising a unit dosage of EPO, and a unit dosage of $\alpha$-MSH or of an $\alpha$-MSH equivalent including the sequence His-Phe-Arg and acting on a melanocortin type 1 receptor, together with a suitable pharmaceutical carrier.

**14.** A pharmaceutical composition according to claim 13, which comprises a unit dosage of EPO and a unit dosage of $\alpha$-MSH.

**15.** A pharmaceutical composition according to claim 13, which comprises a unit dosage of EPO and a unit dosage of an $\alpha$-MSH equivalent including the sequence His-Phe-Arg and acting on a melanocortin type 1 receptor.

**Patentansprüche**

**1.** Anwendung von EPO, eventuell in Kombination mit $\alpha$-MSH oder einem $\alpha$-MSH-Äquivalent, hierunter die Sequenz His-Phe-Arg, und wirkend auf einen Melanocortin-Typ 1-Rezeptor zur Herstellung eines Medikaments zur Behandlung oder Verhinderung eines akuten Entzündungszustands oder einer akuten entzündlichen Erkrankung unter nicht-ischämischen Zuständen in Atemwegen und Lungen oder in der Niere oder in den Harnwegen.

**2.** Anwendung nach Anspruch 1 von EPO zur Herstellung eines Medikaments zur Behandlung oder Verhinderung eines akuten Entzündungszustands oder einer akuten entzündlichen Erkrankung unter nicht-ischämischen Zuständen in Atemwegen und Lungen oder in der Niere oder in den Harnwegen.

**3.** Anwendung nach Anspruch 1 von EPO in Kombination mit $\alpha$-MSH zur Herstellung eines Medikaments zur Behandlung oder Verhinderung eines akuten Entzündungszustands oder einer akuten entzündlichen Erkrankung unter nicht-ischämischen Zuständen in Atemwegen und Lungen oder in der Niere oder in den Harnwegen.

4. Anwendung nach Anspruch 1 von EPO in Kombination mit einem α-MSH-Äquivalent, hierunter die Sequenz His-Phe-Arg, und wirkend auf einen Melanocortin-Typ 1-Rezeptor zur Herstellung eines Medikaments zur Behandlung oder Verhinderung eines akuten Entzündungszustands oder einer akuten entzündlichen Erkrankung unter nicht-ischämischen Zuständen in Atemwegen und Lungen oder in der Niere oder in den Harnwegen.

5. Anwendung nach Anspruch 1-4, worin der akute Entzündungszustand von einer Infektion verursacht wird.

6. Anwendung nach Anspruch 1-5, worin der akute Entzündungszustand von einer Nebenwirkung von Arzneimitteln oder von Vergiftung verursacht wird.

7. Anwendung nach irgendeinem der Ansprüche 1-5, worin der akute Entzündungszustand in Atemwegen und Lungen eine Verschlimmerung von chronisch obstruktive Atemwegserkrankung (COPD) ist.

8. Anwendung nach Anspruch 1-5, worin der akute Entzündungszustand Asthma ist.

9. Anwendung nach irgendeinem der Ansprüche 1-5, worin der akute Entzündungszustand in der Niere oder in den Harnwegen Obstruktion der Harnwege oder Zystitis ist.

10. Anwendung nach irgendeinem der vorhergehenden Ansprüche, worin das Medikament prophylaktisch verabreicht werden soll, um die Etablierung oder den Fortschritt des Zustands oder irgendein Symptom des Zustands zu verhindern.

11. Anwendung nach irgendeinem der Ansprüche 3-10, worin EPO und α-MSH oder ein α-MSH-Äquivalent, hierunter die Sequenz His-Phe-Arg, und einwirkend auf einen Melanocortin-Typ 1-Rezeptor, unabhängig voneinander zu verabreichen sind.

12. Anwendung nach irgendeinem der Ansprüche 3-10, worin EPO und α-MSH oder ein α-MSH-Äquivalent, hierunter die Sequenz His-Phe-Arg, und einwirkend auf einen Melanocortin-Typ 1-Rezeptor, zusammen zu verabreichen sind.

13. Pharmazeutisches Präparat, das eine Dosierungseinheit von EPO und eine Dosierungseinheit von α-MSH oder eines α-MSH-Äquivalents, hierunter die Sequenz His-Phe-Arg, und die auf einen Melanocortin-Typ 1-Rezeptor einwirkt, umfasst, zusammen mit einem geeigneten pharmazeutischen Träger.

14. Pharmazeutisches Präparat nach Anspruch 11, das eine Dosierungseinheit von EPO und eine Dosierungseinheit von α-MSH umfasst.

15. Pharmazeutisches Präparat nach Anspruch 11, das eine Dosierungseinheit von EPO und eine Dosierungseinheit eines α-MSH-Äquivalents, hierunter die Sequenz His-Phe-Arg, und einwirkend auf einen Melanocortin-Typ 1-Rezeptor, umfasst.

**Revendications**

1. Utilisation de l'EPO, optionnellement combiné avec α-MSH ou un équivalent de α-MSH comprenant la séquence His-Phe-Arg et agissant sur un récepteur de la mélanocortine de type 1 pour la préparation d'un médicament pour le traitement ou la prévention d'une affection inflammatoire aigüe ou d'une maladie dans le cadre des pathologies non ischémiques dans la voie respiratoire et dans les poumons, ou dans les reins ou dans l'urètre.

2. Utilisation selon la revendication 1 de l'EPO pour la préparation d'un médicament pour le traitement ou la prévention d'une affection inflammatoire aigüe ou d'une maladie dans le cadre des pathologies non ischémiques dans la voie respiratoire et dans les poumons, ou dans les reins ou dans l'urètre.

3. Utilisation selon la revendication 1 de l'EPO combiné avec α-MSH pour la préparation d'un médicament pour le traitement ou la prévention d'une affection inflammatoire aigüe ou d'une maladie dans le cadre des pathologies non ischémiques dans la voie respiratoire et dans les poumons, ou dans les reins ou dans l'urètre.

**4.** Utilisation selon la revendication 1 de l'EPO combiné avec un équivalent de α-MSH comprenant la séquence His-Phe-Arg et agissant sur un récepteur de la mélanocortine de type 1 pour la préparation d'un médicament pour le traitement ou la prévention d'une affection inflammatoire aigüe ou d'une maladie dans le cadre des pathologies non ischémiques dans la voie respiratoire et dans les poumons, ou dans les reins ou dans l'urètre.

**5.** Utilisation selon l'une quelconque des revendications 1 à 4, dans laquelle l'affection inflammatoire aigüe est causée par une infection.

**6.** Utilisation selon l'une quelconque des revendications 1 à 5, dans laquelle l'affection inflammatoire aigüe est causée par un effet secondaire de médicament ou d'intoxication.

**7.** Utilisation selon l'une quelconque des revendications 1 à 5, dans laquelle l'affection inflammatoire aigüe dans la voie respiratoire et dans les poumons est une exacerbation de la maladie pulmonaire obstructive chronique (MPOC).

**8.** Utilisation selon l'une quelconque des revendications 1 à 5, dans laquelle l'affection inflammatoire aiguë est l'asthme.

**9.** Utilisation selon l'une quelconque des revendications 1 à 5, dans laquelle l'affection inflammatoire aiguë dans les reins ou dans l'urètre est l'obstruction de l'urètre ou la cystite.

**10.** Utilisation selon l'une quelconque des revendications précédentes, dans laquelle le médicament est à administrer de manière prophylactique pour la prévention de l'établissement ou le progrès de l'affection ou d'un symptôme quelconque de l'affection.

**11.** Utilisation selon l'une quelconque des revendications 3 à 10, dans laquelle l'EPO et α-MSH ou un équivalent de α-MSH comprenant la séquence His-Phe-Arg et agissant sur un récepteur de la mélanocortine de type 1 sont à administrer indépendamment l'un de l'autre.

**12.** Utilisation selon l'une quelconque des revendications 3 à 10, dans laquelle l'EPO et α-MSH ou un équivalent de α-MSH comprenant la séquence His-Phe-Arg et agissant sur un récepteur de la mélanocortine de type 1 sont à co-administrer.

**13.** Composition pharmaceutique comprenant un dosage unitaire d'EPO et un dosage unitaire de α-MSH ou d'un équivalent de α-MSH comprenant la séquence His-Phe-Arg et agissant sur un récepteur de la mélanocortine de type 1, avec un porteur pharmaceutiquement approprié.

**14.** Composition pharmaceutique selon la revendication 11, comprenant un dosage unitaire d'EPO et un dosage unitaire de α-MSH.

**15.** Composition pharmaceutique selon la revendication 11, comprenant un dosage unitaire d'EPO et un dosage unitaire d'un équivalent de α-MSH comprenant la séquence His-Phe-Arg et agissant sur un récepteur de la mélanocortine de type 1.

**Fig. 1**

**A**

**B**

Fig. 2

Fig. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 972522 A
- WO 8704623 A
- WO 8800833 A
- WO 9957148 A
- WO 9921571 A
- WO 9641815 A
- US 5028592 A
- US 5731408 A
- US 5830994 A

### Non-patent literature cited in the description

- **LIPTON ; CATANIA.** *Immunol.Today,* 1997, vol. 18, 140-145
- **WONG, K.Y. et al.** *Neuroimmunomodulation,* 1997, vol. 4, 37-41
- **RAJORA, N. et al.** *Peptides,* 1997, vol. 18, 381-385
- **STAR, R.A. et al.** *Proc.Natl.Acad.Sci.U.S.A,* 1995, vol. 92, 8016-8020
- **LUGER, T.A.** *J.Investig.Dermatol.Symp.Proc.,* 1997, vol. 2, 87-93
- **HARTMEYER, M.** *J.Immunol.,* 1997, vol. 159, 1930-1937
- **SADAMOTO, Y. et al.** *Biochem. Biophys. Res. Commun.,* 1998, vol. 253, 26-32
- **SAKANAKA, M. et al.** *Proc.Natl.Acad.Sci.U.S.A,* 1998, vol. 95, 4635-4640
- **BERNAUDIN, M. et al.** *J.Cereb.Blood Flow Metab,* 1999, vol. 19, 643-651
- Remington: The science and practice of pharmacy. Mack Publishing, 2000
- Encyclopedia of Pharmaceutical Technology. Marcel Dekker, Inc, 1988
- **APOSTOL, E. ; C. A. ECELBARGER ; J. TERRIS ; A. D. BRADFORD ; P. ANDREWS ; M. A. KNEPPER.** Reduced renal medullary water channel expression in puromycin aminonucleoside-induced nephrotic syndrome. *J.Am.Soc.Nephrol.,* 1997, vol. 8, 15-24
- **BERENS, K. L. ; R. R. VERANI ; D. R. LUKE.** Role of neutrophils and macrophages in experimental nephrosis of the rat. *Ren Fail.,* 1998, vol. 20, 53-63
- **BERNAUDIN, M. ; H. H. MARTI ; S. ROUSSEL ; D. DIVOUX ; A. NOUVELOT ; E. T. MACKENZIE ; E. PETIT.** A potential role for erythropoietin in focal permanent cerebral ischemia in mice. *J.Cereb.Blood Flow Metab,* 1999, vol. 19, 643-651
- **BHARDWAJ, R. ; J. BLANCHARD.** Controlled-release delivery system for the alpha-MSH analog melanotan-I using poloxamer 407. *J.Pharm.Sci.,* 1996, vol. 85, 915-919
- **BOHLE, A. ; S. MACKENSEN-HAEN ; H. VON GISE.** Significance of tubulointerstitial changes in the renal cortex for the excretory function and concentration ability of the kidney: a morphometric contribution. *Am.J.Nephrol.,* 1987, vol. 7, 421-433
- **BORGHI, L. ; T. MESCHI ; F. AMATO ; A. NOVARINI ; A. GIANNINI ; C. QUARANTELLI ; F. MINEO.** Nifedipine and methylprednisolone in facilitating ureteral stone passage: a randomized, double-blind, placebo-controlled study [see comments. *J.Urol.,* 1994, vol. 152, 1095-1098
- **BUNN, H. F. ; J. GU ; L. E. HUANG ; J. W. PARK ; H. ZHU.** Erythropoietin: a model system for studying oxygen-dependent gene regulation. *J.Exp.Biol.,* 1998, vol. 201 (8), 1197-1201
- **CANBOLAT, O. ; J. FANDREY ; W. JELKMANN.** Effects of modulators of the production and degradation of hydrogen peroxide on erythropoietin synthesis. *Respir.Physiol,* 1998, vol. 114, 175-183
- **CATANIA, A. ; N. RAJORA ; F. CAPSONI ; F. MINONZIO ; R. A. STAR ; J. M. LIPTON.** The neuropeptide alpha-MSH has specific receptors on neutrophils and reduces chemotaxis in vitro. *Peptides,* 1996, vol. 17, 675-679
- **CHHAJLANI, V. ; J. E. WIKBERG.** Molecular cloning and expression of the human melanocyte stimulating hormone receptor cDNA. *FEBS Lett.,* 1992, vol. 309, 417-420
- **CHHAJLANI, V. ; R. MUCENIECE ; J. E. WIKBERG.** Molecular cloning of a novel human melanocortin receptor. *Biochem Biophys Res Commun,* 17 January 1996, vol. 218 (2), 638
- *Biochem.Biophys.Res.Commun.,* 1993, vol. 195, 866-873

- **CHYE, R. ; N. LICKISS.** he use of corticosteroids in the management of bilateral malignant ureteric obstruction. *J.Pain Symptom.Manage,* 1994, vol. 9, 537-540
- **DIAMOND, J. R. ; D. KEES-FOLTS ; G. DING ; J. E. FRYE ; N. C. RESTREPO.** Macrophages, monocyte chemoattractant peptide-1, and TGF-beta 1 in experimental hydronephrosis. *Am.J.Physiol,* 1994, vol. 266, F926-F933
- **DIGICAYLIOGLU, M. ; S. BICHET ; H. H. MARTI ; R. H. WENGER ; L. A. RIVAS ; C. BAUER ; M. GASSMANN.** Localization of specific erythropoietin binding sites in defined areas of the mouse brain. *Proc.Natl.Acad.Sci.U.S.A,* 1995, vol. 92, 3717-3720
- **EHLEBEN, W. ; T. PORWOL ; J. FANDREY ; W. KUMMER ; H. ACKER.** Cobalt and desferrioxamine reveal crucial members of the oxygen sensing pathway in HepG2 cells. *Kidney Int.,* 1997, vol. 51, 483-491
- **EL NAHAS, A. M.** Pathways to renal fibrosis. *Exp.Nephrol.,* 1995, vol. 3, 71-75
- **FANDREY, J. ; H. F. BUNN.** In vivo and in vitro regulation of erythropoietin mRNA: measurement by competitive polymerase chain reaction. *Blood,* 1993, vol. 81, 617-623
- **FANDREY, J. ; S. FREDE ; W. JELKMANN.** Role of hydrogen peroxide in hypoxia-induced erythropoietin production. *Biochem.J.,* 1994, vol. 303 (2), 507-510
- **FANDREY, J. ; S. FREDE ; W. EHLEBEN ; T. PORWOL ; H. ACKER ; W. JELKMANN.** Cobalt chloride and desferrioxamine antagonize the inhibition of erythropoietin production by reactive oxygen species. *Kidney Int,* 1997, vol. 51, 492-496
- **FERNANDEZ-LLAMA, P. ; P. ANDREWS ; S. NIELSEN ; C. A. ECELBARGER ; M. A. KNEPPER.** Impaired aquaporin and urea transporter expression in rats with adriamycin-induced nephrotic syndrome. *Kidney Int.,* 1998, vol. 53, 1244-1253
- **FERNANDEZ-LLAMA, P. ; P. ANDREWS ; R. TURNER ; S. SAGGI ; J. DIMARI ; T. H. KWON ; S. NIELSEN ; R. SAFIRSTEIN ; M. A. KNEPPER.** Decreased abundance of collecting duct aquaporins in post-ischemic renal failure in rats. *J.Am.Soc.Nephrol.,* 1999, vol. 10, 1658-1668
- **FROKIAER, J. ; A. S. NIELSEN ; L. KNUDSEN ; J. C. DJURHUUS ; E. B. PEDERSEN.** The effect of indomethacin infusion on renal hemodynamics and on the renin-angiotensin system during unilateral ureteral obstruction of the pig. *J.Urol.,* 1993, vol. 150, 1557-1563
- **GOLDBERG, M. A. ; G. A. GLASS ; J. M. CUNNINGHAM ; H. F. BUNN.** The regulated expression of erythropoietin by two human hepatoma cell lines. *Proc. Natl.Acad.Sci. U. S.A,* 1987, vol. 84, 7972-7976
- **GOLDBERG, M. A. ; S. P. DUNNING ; H. F. BUNN.** Regulation of the erythropoietin gene: evidence that the oxygen sensor is a heme protein. *Science,* 1988, vol. 242, 1412-1415
- **GOLDBERG, M. A. ; C. C. GAUT ; H. F. BUNN.** Erythropoietin mRNA levels are governed by both the rate of gene transcription and posttranscriptional events. *Blood,* 1991, vol. 77, 271-277
- **GORLACH, A. ; J. FANDREY ; G. HOLTERMANN ; H. ACKER.** Effects of cobalt on haem proteins of erythropoietin-producing HepG2 cells in multicellular spheroid culture. *FEBS Lett.,* 1994, vol. 348, 216-218
- **HARTMEYER, M. ; T. SCHOLZEN ; E. BECHER ; R. S. BHARDWAJ ; T. SCHWARZ ; T. A. LUGER.** Human dermal microvascular endothelial cells express the melanocortin receptor type 1 and produce increased levels of IL-8 upon stimulation with alpha-melanocyte-stimulating hormone. *J.Immunol.,* 1997, vol. 159, 1930-1937
- **ISHIBASHI, K. ; S. SASAKI ; K. FUSHIMI ; T. YAMAMOTO ; M. KUWAHARA ; F. MARUMO.** Immunolocalization and effect of dehydration on AQP3, a basolateral water channel of kidney collecting ducts. *Am.J.Physiol,* 1997, vol. 272, F235-F241
- **ISHIDOYA, S. ; J. MORRISSEY ; R. MCCRACKEN ; A. REYES ; S. KLAHR.** Angiotensin II receptor antagonist ameliorates renal tubulointerstitial fibrosis caused by unilateral ureteral obstruction. *Kidney Int.,* 1995, vol. 47, 1285-1294
- **JAENIKE, J. R.** The renal functional defect of postobstructive nephyropathy. The effects of bilateral ureteral obstruction in the rat. *J.Clin.Invest,* 1972, vol. 51, 2999-3006
- **JELKMANN, W.** Erythropoietin: structure, control of production, and function. *Physiol Rev.,* 1992, vol. 72, 449-489
- **JONES, E. A. ; A. SHAHED ; D. A. SHOSKES.** Modulation of apoptotic and inflammatory genes by bioflavonoids and angiotensin II inhibition in ureteral obstruction [In Process Citation. *Urology,* 2000, vol. 56, 346-351
- **JONES, E. A. ; D. A. SHOSKES.** The effect of mycophenolate mofetil and polyphenolic bioflavonoids on renal ischemia reperfusion injury and repair. *J.Urol.,* 2000, vol. 163, 999-1004
- **KANETO, H. ; J. MORRISSEY ; S. KLAHR.** Increased expression of TGF-beta 1 mRNA in the obstructed kidney of rats with unilateral ureteral ligation. *Kidney Int.,* 1993, vol. 44, 313-321
- **KANETO, H. ; J. MORRISSEY ; R. MCCRACKEN ; A. REYES ; S. KLAHR.** Enalapril reduces collagen type IV synthesis and expansion of the interstitium in the obstructed rat kidney. *Kidney Int.,* 1994, vol. 45, 1637-1647

- **KLAHR, S. ; J. J. MORRISSEY.** The role of growth factors, cytokines, and vasoactive compounds in obstructive nephropathy. *Semin.Nephrol.,* 1998, vol. 18, 622-632
- **KLAHR, S. ; J. MORRISSEY.** Angiotensin II and gene expression in the kidney. *Am.J.Kidney Dis.,* 1998, vol. 31, 171-176
- **KRAMER, B. K ; M. BUCHER ; P. SANDNER ; K. P. ITTNER ; G. A. RIEGGER ; T. RITTHALER ; A. KURTZ.** Effects of hypoxia on growth factor expression in the rat kidney in vivo. *Kidney Int.,* 1997, vol. 51, 444-447
- **KUNCIO, G. S. ; E. G. NEILSON ; T. HAVERTY.** Mechanisms of tubulointerstitial fibrosis. *Kidney Int.,* 1991, vol. 39, 550-556
- **KWON, T. H. ; J. FROKIAER ; P. FERNANDEZ-LLAMA ; M. A. KNEPPER ; S. NIELSEN.** Reduced abundance of aquaporins in rats with bilateral ischemia-induced acute renal failure: prevention by alpha-MSH. *Am.J.Physiol,* 1999, vol. 277, F413-F427
- **LIPTON, J. M. ; A. CATANIA.** Anti-inflammatory actions of the neuroimmunomodulator alpha-MSH. *Immunol. Today,* 1997, vol. 18, 140-145
- **LUGER, T. A. ; T. SCHOLZEN ; S. GRABBE.** The role of alpha-melanocyte-stimulating hormone in cutaneous biology. *J.Investig.Dermatol.Symp.Proc.,* 1997, vol. 2, 87-93
- **MARTI, H. H. ; R. H. WENGER ; L. A. RIVAS ; U. STRAUMANN ; M. DIGICAYLIOGLU ; V. HENN ; Y. YONEKAWA ; C. BAUER ; M. GASSMANN.** Erythropoietin gene expression in human, monkey and murine brain. *Eur.J.Neurosci.,* 1996, vol. 8, 666-676
- **MORISHITA, E. ; S. MASUDA ; M. NAGAO ; Y. YASUDA ; R. SASAKI.** Erythropoietin receptor is expressed in rat hippocampal and cerebral cortical neurons, and erythropoietin prevents in vitro glutamate-induced neuronal death. *Neuroscience,* 1997, vol. 76, 105-116
- **MORIYAMA, T. ; N. KAWADA ; A. ANDO ; A. YAMAUCHI ; M. HORIO ; K. NAGATA ; E. IMAI ; M. HORI.** Up-regulation of HSP47 in the mouse kidneys with unilateral ureteral obstruction. *Kidney Int.,* 1998, vol. 54, 110-119
- **MORRISSEY, J. J. ; S. KLAHR.** Rapid communication. Enalapril decreases nuclear factor kappa B activation in the kidney with ureteral obstruction. *Kidney Int.,* 1997, vol. 52, 926-933
- **MORRISSEY, J. J. ; S. KLAHR.** Differential effects of ACE and AT1 receptor inhibition on chemoattractant and adhesion molecule synthesis. *Am.J.Physiol,* 1998, vol. 274, F580-F586
- **MORRISSEY, J. J. ; S. KLAHR.** Effect of AT2 receptor blockade on the pathogenesis of renal fibrosis. *Am.J.Physiol,* 1999, vol. 276, F39-F45
- **MOUNTJOY, K. G. ; L. S. ROBBINS ; M. T. MORTRUD ; R. D. CONE.** The cloning of a family of genes that encode the melanocortin receptors. *Science,* 1992, vol. 257, 1248-1251
- **NAGLE, R. B. ; M. R. KNEISER ; R. E. BULGER ; E. P. BENDITT.** Induction of smooth muscle characteristics in renal interstitial fibroblasts during obstructive nephropathy. *Lab Invest,* 1973, vol. 29, 422-427
- **NIELSEN, S. ; S. R. DIGIOVANNI ; E. I. CHRISTENSEN ; M. A. KNEPPER ; H. W. HARRIS.** Cellular and subcellular immunolocalization of vasopressin-regulated water channel in rat kidney. *Proc.Natl.Acad.Sci.U.S.A,* 1993, vol. 90, 11663-11667
- **NIELSEN, S. ; C. L. CHOU ; D. MARPLES ; E. I. CHRISTENSEN ; B. K. KISHORE ; M. A. KNEPPER.** Vasopressin increases water permeability of kidney collecting duct by inducing translocation of aquaporin-CD water channels to plasma membrane. *Proc.Natl.Acad.Sci.U.S.A,* 1995, vol. 92, 1013-1017
- **PERLMUTTER, A. ; L. MILLER ; L. A. TRIMBLE ; D. N. MARION ; E. D. VAUGHAN, JR. ; D. FELSEN. TORADOL.** an NSAID used for renal colic, decreases renal perfusion and ureteral pressure in a canine model of unilateral ureteral obstruction. *J.Urol.,* 1993, vol. 149, 926-930
- **PETERSEN, J. S. ; M. SHALMI ; H. R. LAM ; S. CHRISTENSEN.** Renal response to furosemide in conscious rats: effects of acute instrumentation and peripheral sympathectomy. *J.Pharmacol.Exp.Ther.,* 1991, vol. 258, 1-7
- **PORWOL, T. ; W. EHLEBEN ; K ZIEROLD ; J. FANDREY ; H. ACKER.** The influence of nickel and cobalt on putative members of the oxygen- sensing pathway of erythropoietin-producing HepG2 cells. *Eur.J.Biochem.,* 1998, vol. 256, 16-23
- **RAJORA, N. ; G. BOCCOLI ; D. BURNS ; S. SHARMA ; A P. CATANIA ; J. M. LIPTON.** alpha-MSH modulates local and circulating tumor necrosis factor-alpha in experimental brain inflammation. *J.Neurosci.,* 1997, vol. 17, 2181-2186
- **RAJORA, N. ; G. BOCCOLI ; A. CATANIA ; J. M. LIPTON.** alpha-MSH modulates experimental inflammatory bowel disease. *Peptides,* 1997, vol. 18, 381-385
- **RATCLIFFE, P. J. ; P. H. MAXWELL ; C. W. PUGH.** Beyond erythropoietin: the oxygen sensor. *Nephrol.Dial.Transplant,* 1997, vol. 12, 1842-1848
- **RATCLIFFE, P. J. ; B. L. EBERT ; J. D. FIRTH ; J. M. GLEADLE ; P. H. MAXWELL ; M. NAGAO ; J. F. O'ROURKE ; C. W. PUGH ; S. M. WOOD.** Oxygen regulated gene expression: erythropoietin as a model system. *Kidney Int.,* 1997, vol. 51, 514-526

- **RICARDO, S. D. ; M. E. LEVINSON ; M. R. DEJOSEPH ; J. R. DIAMOND.** Expression of adhesion molecules in rat renal cortex during experimental hydronephrosis. *Kidney Int.,* 1996, vol. 50, 2002-2010
- **SABOLIC, I. ; T. KATSURA ; J. M. VERBAVATZ ; D. BROWN.** The AQP2 water channel: effect of vasopressin treatment, microtubule disruption, and distribution in neonatal rats. *J Membr Biol,* May 1995, vol. 145 (1), 107-8
- *J.Membr.Biol.,* 1995, vol. 143, 165-175
- **SADAMOTO, Y. ; K. IGASE ; M. SAKANAKA ; K. SATO ; H. OTSUKA ; S. SAKAKI ; S. MASUDA ; R. SASAKI.** Erythropoietin prevents place navigation disability and cortical infarction in rats with permanent occlusion of the middle cerebral artery. *Biochem.Biophys.Res.Commun.,* 1998, vol. 253, 26-32
- **SAKANAKA, M. ; T. C. WEN ; S. MATSUDA ; S. MASUDA ; E. MORISHITA ; M. NAGAO ; R. SASAKI.** In vivo evidence that erythropoietin protects neurons from ischemic damage. *Proc.Natl.Acad.Sci.U.S.A,* 1998, vol. 95, 4635-4640
- **SALCEDA, S. ; J. CARO.** Hypoxia-inducible factor 1alpha (HIF-1alpha) protein is rapidly degraded by the ubiquitin-proteasome system under normoxic conditions. Its stabilization by hypoxia depends on redox-induced changes. *J.Biol.Chem.,* 1997, vol. 272, 22642-22647
- **SCHIOTH, H. B. ; V. CHHAJLANI ; R. MUCENIECE ; V. KLUSA ; J. E. WIKBERG.** Major pharmacological distinction of the ACTH receptor from other melanocortin receptors. *Life Sci.,* 1996, vol. 59, 797-801
- **SCHUSTER, S. J. ; E. V. BADIAVAS ; P. COSTA-GIOMI ; R. WEINMANN ; A. J. ERSLEV ; J. CARO.** Stimulation of erythropoietin gene transcription during hypoxia and cobalt exposure. *Blood,* 1989, vol. 73, 13-16
- **SEMENZA, G. L. ; G. L. WANG.** A nuclear factor induced by hypoxia via de novo protein synthesis binds to the human erythropoietin gene enhancer at a site required for transcriptional activation. *Mol.Cell Biol.,* 1992, vol. 12, 5447-5454
- **SHARMA, A. K. ; S. M. MAUER ; Y. KIM ; A. F. MICHAEL.** Interstitial fibrosis in obstructive nephropathy. *Kidney Int.,* 1993, vol. 44, 774-788
- **SONNENBERG, H. ; D. R. WILSON.** The role of the medullary collecting ducts in postobstructive diuresis. *J.Clin.Invest,* 1976, vol. 57, 1564-1574
- **SQUADRITO, F. ; S. GUARINI ; D. ALTAVILLA ; G. SQUADRITO ; G. M. CAMPO ; M. ARLOTTA ; C. QUARTARONE ; A. SAITTA ; D. CUCINOTTA ; C. BAZZANI.** Adrenocorticotropin reverses vascular dysfunction and protects against splanchnic artery occlusion shock. *Br.J.Pharmacol.,* 1999, vol. 128, 816-822
- **SQUADRITO, F. ; D. ALTAVILLA ; G. SQUADRITO ; G. M. CAMPO ; M. ARLOTTA ; C. QUARTARONE ; A. SAITTA ; A. P. CAPUTI.** Recombinant human erythropoietin inhibits iNOS activity and reverts vascular dysfunction in splanchnic artery occlusion shock. *Br.J.Pharmacol.,* 1999, vol. 127, 482-488
- **STAR, R. A. ; N. RAJORA ; J. HUANG ; R. C. STOCK ; A. CATANIA ; J. M. LIPTON.** Evidence of autocrine modulation of macrophage nitric oxide synthase by alpha-melanocyte-stimulating hormone. *Proc.Natl.Acad.Sci.U.S.A,* 1995, vol. 92, 8016-8020
- **STRUTZ, F. ; E. G. NEILSON.** The role of lymphocytes in the progression of interstitial disease. *Kidney Int.Suppl,* 1994, vol. 45, S106-S110
- **STRUTZ, F.** Novel aspects of renal fibrogenesis. *Nephrol.Dial.Transplant.,* 1995, vol. 10, 1526-1532
- **STRUTZ, F. ; G. A. MULLER.** On the progression of chronic renal disease. *Nephron,* 1995, vol. 69, 371-379
- **TAN, C. C. ; K U. ECKARDT ; J. D. FIRTH ; P. J. RATCLIFFE.** Feedback modulation of renal and hepatic erythropoietin mRNA in response to graded anemia and hypoxia. *Am.J.Physiol,* 1992, vol. 263, F474-F481
- **VAN DE MERWE, J. P. ; H. J. ARENDSEN.** Interstitial cystitis: a review of immunological aspects of the aetiology and pathogenesis, with a hypothesis. *BJU.Int.,* 2000, vol. 85, 995-999
- **VANETTI, M. ; C. SCHONROCK ; W. MEYERHOF ; V. HOLLT.** Molecular cloning of a bovine MSH receptor which is highly expressed in the testis. *FEBS Lett.,* 1994, vol. 348, 268-272
- **WANG, G. L. ; G. L. SEMENZA.** General involvement of hypoxia-inducible factor 1 in transcriptional response to hypoxia. *Proc.Natl.Acad.Sci.U.S.A,* 1993, vol. 90, 4304-4308
- **WANG, G. L. ; G. L. SEMENZA.** Desferrioxamine induces erythropoietin gene expression and hypoxia-inducible factor 1 DNA-binding activity: implications for models of hypoxia signal transduction. *Blood,* 1993, vol. 82, 3610-3615
- **WANG, G. L. ; B. H. JIANG ; G. L. SEMENZA.** Effect of altered redox states on expression and DNA-binding activity of hypoxia-inducible factor 1. *Biochem.Biophys.Res.Commun.,* 1995, vol. 212, 550-556
- **WENGER, R. H. ; I. KVIETIKOVA ; A. ROLFS ; M. GASSMANN ; H. H. MARTI.** Hypoxia-inducible factor-1 alpha is regulated at the post-mRNA level. *Kidney Int.,* 1997, vol. 51, 560-563

- **WONG, K. Y. ; N. RAJORA ; G. BOCCOLI ; A. CATANIA ; J. M. LIPTON.** A potential mechanism of local anti-inflammatory action of alpha- melanocyte-stimulating hormone within the brain: modulation of tumor necrosis factor-alpha production by human astrocytic cells. *Neuroimmunomodulation,* 1997, vol. 4, 37-41

- **XIA, Y. ; J. E. WIKBERG ; V. CHHAJLANI.** Expression of melanocortin 1 receptor in periaqueductal gray matter. *Neuroreport,* 1995, vol. 6, 2193-2196
- **ZANJANI, E. D. ; J. POSTER ; H. BURLINGTON ; L. I. MANN ; L. R. WASSERMAN.** Liver as the primary site of erythropoietin formation in the fetus. *J.Lab Clin.Med.,* 1977, vol. 89, 640-644